# EUROPEAN PATENT SPECIFICATION

(11) **EP 0 646 015 B1**
(45) Date of publication and mention of the grant of the patent: **07.01.1999**
(21) Application number: 93915326.8
(22) Date of filing: 10.06.1993
(51) Int. Cl.: A61K 39/00, C12N 15/12, C07K 14/705, C07K 7/06

(54) **CONTRACEPTIVE VACCINE**
EMPFÄNGNISVERHÜTENDER IMPFSTOFF
VACCIN CONTRACEPTIF

(30) Priority: 12.06.1992 US 897883
(43) Date of publication of application: 05.04.1995
(73) Proprietor: THE UNIVERSITY OF CONNECTICUT, Storrs, CT 06269-1133 (US)
(72) Inventor: PRIMAKOFF, Paul, West Simsbury, CT 06092 (US); MYLES, Diana, G., West Simsbury, CT 06092 (US)
(74) Representative: Kirkham, Nicholas Andrew
(86) International application number: US9305640
(87) International publication number: WO9325233

(56) References cited:
- WO-A-92/10569
- WO-A-93/06859
- NATURE. vol. 356, 19 March 1992, LONDON GB pages 248 - 252 C.P. BLOBEL ET AL. 'A POTENTIAL FUSION PEPTIDE AND AN INTEGRIN LIGAND DOMAIN IN A PROTEIN ACTIVE IN SPERM-EGG FUSION.' cited in the application
- NATURE. vol. 335, 6 October 1988, LONDON GB pages 543 - 546 P. PRIMAKOFF ET AL. 'FULLY EFFECTIVE CONTRACEPTION IN MALE AND FEMALE GUINEA PIGS IMMUNIZED WITH THE SPERM PROTEIN PH-20.'
- MOLECULAR BIOLOGY OF THE CELL (AM. SOC. CELL BIOL., 32ND ANNUAL MEETING, NOVEMBER 15-19, 1992, DENVER, COLORADO, US) vol. 3, no. SUPL, September 1992, BETHESDA, MD.,US page 212A D.G. MILES ET AL. 'INHIBITION OF SPERM-EGG FUSION BY PEPTIDES CONTAINING THE TDE SEQUENCE IN THE DISINTEGRIN DOMAIN OF THE GUINEA PIG SPERM PROTEIN PH-30.'

SEQUENCE LISTING

(1) GENERAL INFORMATION:

(i) APPLICANT:
(A) ADDRESSEE: University of Connecticut
(B) STREET: 213 Whetten Graduate Center
438 Whitney Road Extension
(C) CITY: Storrs
(D) STATE: CT
(E) COUNTRY: USA
(F) POSTAL CODE (ZIP): 06269

(ii) TITLE OF INVENTION: Contraceptive Vaccine

(iii) NUMBER OF SEQUENCES: 46

(iv) CORRESPONDENCE ADDRESS:
(A) ADDRESSEE: Hamilton, Brook, Smith & Reynolds, P.C.
(B) STREET: Two Militia Drive
(C) CITY: Lexington
(D) STATE: MA
(E) COUNTRY: USA
(F) ZIP: 02173

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER: 07/897,883
(B) FILING DATE: June 12, 1992
(C) CLASSIFICATION:

(viii) ATTORNEY/AGENT INFORMATION:
(A) NAME: Brook, David E.
(B) REGISTRATION NUMBER: 22,592
(C) REFERENCE/DOCKET NUMBER: UCT90-01AA

(ix) TELECOMMUNICATION INFORMATION:
(A) TELEPHONE: (617) 861-6240
(B) TELEFAX: (617) 861-9540

(2) INFORMATION FOR SEQ ID NO:1:

(i) SEQUENCE CHARACTERISTICS:
(A) LENGTH: 2152 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: CDS
(B) LOCATION: 355..1941

(xi) SEQUENCE DESCRIPTION: SEQ ID NO:1:

GCTTTACTGT GAGGTTGCTT GTACATTGAT TTTCCAGTTC TCTTAAGAAT CTGTGGCTTG 60

ATGTAGCTCA CACGAATCCA GGAGGATTTT TGTTTCTTAA TTTTGATGAC TGCGTACATG 120

ATTAGTAGTA CATCGTAAAG TCTCTTCCAA CAAGTTACAG ATGGTGCAAC ATTCAAAACA 180

TTCCTGAAAT ACAAAACAAG AAGAATATTT TAATGTAACA GAGTTGTTTA CCTCTTTATC 240

CACCAAAGTG ACCTCACTGT ACTACGCTTC TTTTGGGCTC ATATTGTGCA ACAAATATTG 300

GAAAAAACAG TGTATAAGAA GAAAAAGTAT TTTTCACAGC TGTTACTCTT TCTA ATG 357
Met
1

GGA GCA TTC ACT TTT AAA CAC AGC TTT TTT GGG AGT TTT GTT GAG TGC 405
Gly Ala Phe Thr Phe Lys His Ser Phe Phe Gly Ser Phe Val Glu Cys
            5                   10                  15

AGT GGA GTA TTG CAG ACA GTG TTT ATT TTC CTT CTA ATT CCA TGT TGC 453
Ser Gly Val Leu Gln Thr Val Phe Ile Phe Leu Leu Ile Pro Cys Cys
        20                  25                  30

CTG GCT GAT AAA AGG GCA CCA CCA CTC ATC CCA AAT GTG CCT TTG CTC 501
Leu Ala Asp Lys Arg Ala Pro Pro Leu Ile Pro Asn Val Pro Leu Leu
    35                  40                  45

TGG GTC TGG AAT GCC CCA ACT GAA TTT TGT ATA GGA GGA ACC AAT CAA 549
Trp Val Trp Asn Ala Pro Thr Glu Phe Cys Ile Gly Gly Thr Asn Gln
50                  55                  60                  65

CCA CTA GAT ATG AGC TTT TTC TCT ATA GTA GGA ACT CCC AGG AAA AAT 597
Pro Leu Asp Met Ser Phe Phe Ser Ile Val Gly Thr Pro Arg Lys Asn
                70                  75                  80

ATC ACA GGG CAA AGT ATT ACA CTA TAT TAT GTT GAT AGA CTT GGC TAC 645
Ile Thr Gly Gln Ser Ile Thr Leu Tyr Tyr Val Asp Arg Leu Gly Tyr
            85                  90                  95

TAT CCT TAC ATA GAT CCT CAC ACA GGT GCG ATT GTG CAT GGA GGA CTC 693
Tyr Pro Tyr Ile Asp Pro His Thr Gly Ala Ile Val His Gly Gly Leu
        100                 105                 110

CCC CAG TTG ATG AAT TTA CAA CAG CAT TTG AGA AAA TCA AGG CAA GAC 741
Pro Gln Leu Met Asn Leu Gln Gln His Leu Arg Lys Ser Arg Gln Asp
    115                 120                 125

ATT TTA TTT TAC ATG CCC ACA GAC AGC GTG GGC TTG GCT GTC ATT GAC 789
Ile Leu Phe Tyr Met Pro Thr Asp Ser Val Gly Leu Ala Val Ile Asp
130                 135                 140                 145

TGG GAA GAG TGG AGG CCC ACC TGG ACA AGA AAC TGG AGA CCT AAG GAT 837
Trp Glu Glu Trp Arg Pro Thr Trp Thr Arg Asn Trp Arg Pro Lys Asp
                150                 155                 160

ATT TAC AGG AAT AAA TCT ATT GAG TTG GTT AAG AGC CAG CAT CCA CAG 885
Ile Tyr Arg Asn Lys Ser Ile Glu Leu Val Lys Ser Gln His Pro Gln
            165                 170                 175

TAT AAT CAC TCA TAT GCT GTT GCC GTA GCC AAA AGA GAC TTT GAA AGG 933
Tyr Asn His Ser Tyr Ala Val Ala Val Ala Lys Arg Asp Phe Glu Arg
        180                 185                 190

ACA GGG AAG GCT TTC ATG CTA GAA ACT TTA AAA CTG GGA AAA TCA CTT 981
Thr Gly Lys Ala Phe Met Leu Glu Thr Leu Lys Leu Gly Lys Ser Leu
    195                 200                 205

AGG CCA AGT AGC TTA TGG GGT TAT TAT CTT TTT CCT GAT TGC TAC AAC 1029
Arg Pro Ser Ser Leu Trp Gly Tyr Tyr Leu Phe Pro Asp Cys Tyr Asn
210                 215                 220                 225

ACT CAT TTC ACT AAA CCC AAT TAT GAT GGG CAT TGC CCT CCT ATA GAA 1077
Thr His Phe Thr Lys Pro Asn Tyr Asp Gly His Cys Pro Pro Ile Glu
                230                 235                 240

CTG CAA AGA AAT AAC GAT CTC CAA TGG TTG TGG AAC GAC AGC ACT GCC 1125
Leu Gln Arg Asn Asn Asp Leu Gln Trp Leu Trp Asn Asp Ser Thr Ala
            245                 250                 255

CTT TAC CCA TCT GTT TAT TTG ACC AGT CGA GTA AGA TCA TCT CAA AAT 1173
Leu Tyr Pro Ser Val Tyr Leu Thr Ser Arg Val Arg Ser Ser Gln Asn
        260                 265                 270

GGT GCA CTT TAT GTT CGT AAT CGT GTA CAC GAG TCC ATT AGG GTT TCG 1221
Gly Ala Leu Tyr Val Arg Asn Arg Val His Glu Ser Ile Arg Val Ser
    275                 280                 285

AAA CTC ATG GAT GAC AAA AAC CCA CTT CCG ATT TAT GTG TAT ATC CGC 1269
Lys Leu Met Asp Asp Lys Asn Pro Leu Pro Ile Tyr Val Tyr Ile Arg
290                 295                 300                 305

CTC GTT TTT ACC GAT CAA ACT ACT ACA TTC CTT GAA CTG GAT GAT CTT 1317
Leu Val Phe Thr Asp Gln Thr Thr Thr Phe Leu Glu Leu Asp Asp Leu
                310                 315                 320

GTG CAT TCA GTT GGC GAA ATT GTT CCT CTA GGT GTC TCT GGA ATA ATA 1365
Val His Ser Val Gly Glu Ile Val Pro Leu Gly Val Ser Gly Ile Ile
            325                 330                 335

ATA TGG GGA AGT CTT AGT TTA ACA CGA AGT TTG GTT TCT TGT ATA GGA 1413
Ile Trp Gly Ser Leu Ser Leu Thr Arg Ser Leu Val Ser Cys Ile Gly
        340                 345                 350

CTA GAA AAT TAC ATG AAG GGT ACA CTC CTG CCT TAC TTA ATC AAT GTC 1461
Leu Glu Asn Tyr Met Lys Gly Thr Leu Leu Pro Tyr Leu Ile Asn Val
    355                 360                 365

ACC CTA GCA GCC AAA ATG TGT GGC CAA GTG CTT TGT AAG AAT CAA GGA 1509
Thr Leu Ala Ala Lys Met Cys Gly Gln Val Leu Cys Lys Asn Gln Gly
370                 375                 380                 385

ATT TGC ACA AGG AAA GAC TGG AAC ACA AAC ACC TAT CTT CAC CTA AAC 1557
Ile Cys Thr Arg Lys Asp Trp Asn Thr Asn Thr Tyr Leu His Leu Asn
                390                 395                 400

GCA ACA AAT TTT GAC ATT GAA CTT CAG CAA AAT GGG AAG TTT GTA GTA 1605
Ala Thr Asn Phe Asp Ile Glu Leu Gln Gln Asn Gly Lys Phe Val Val
            405                 410                 415

CAT GGA AAA CCA TCA CTT GAA GAC CTG CAG GAA TTT TCC AAA AAT TTT 1653
His Gly Lys Pro Ser Leu Glu Asp Leu Gln Glu Phe Ser Lys Asn Phe
        420                 425                 430

CAT TGC TCC TGT TAT ACC AAT GTG GCT TGT AAG GAC AGA CTT GAT GTA     1701
His Cys Ser Cys Tyr Thr Asn Val Ala Cys Lys Asp Arg Leu Asp Val
    435                 440                 445

CAT AAT GTC CGT TCT GTT AAT GTG TGT ACT GCC AAT AAT ATT TGT ATA     1749
His Asn Val Arg Ser Val Asn Val Cys Thr Ala Asn Asn Ile Cys Ile
450                 455                 460                 465

GAT GCT GTT TTA AAT TTT CCA TCC CTG GAT GAT GAT GAT GAG CCT CCC     1797
Asp Ala Val Leu Asn Phe Pro Ser Leu Asp Asp Asp Asp Glu Pro Pro
                470                 475                 480

ATC ACA GAC GAC ACG TCT CAA AAT CAA GAC AGC ATC TCC GAC ATC ACA     1845
Ile Thr Asp Asp Thr Ser Gln Asn Gln Asp Ser Ile Ser Asp Ile Thr
            485                 490                 495

TCA TCT GCT CCA CCG TCT TCA CAT ATT CTT CCA AAG GAT CTC AGT TGG     1893
Ser Ser Ala Pro Pro Ser Ser His Ile Leu Pro Lys Asp Leu Ser Trp
        500                 505                 510

TGC CTC TTC CTT CTC TCT ATC TTT TCA CAG CAC TGG AAA TAT TTA CTA     1941
Cys Leu Phe Leu Leu Ser Ile Phe Ser Gln His Trp Lys Tyr Leu Leu
    515                 520                 525

TAGGCTCATG GCAACTGAAA AGTACCAAAT TCAATATCAT AAAAATTCTA TAATCAAAAT     2001

CCTTTGAATT TTTAAAGCAA AATACATACT ATTCTATCAA AGACACTGTA AAGCCTGTGG     2061

TACTTGGAAG ATACAGCTTT CTTTTGAGAA GAGTGAAGAT TTGAATAAAA CAAAATTACT     2121

GAAAAAAAAA AAAAAAAAAA AAAAAAAAAA A                                    2152

(2) INFORMATION FOR SEQ ID NO:2:

        (i) SEQUENCE CHARACTERISTICS:
            (A) LENGTH: 529 amino acids
            (B) TYPE: amino acid
            (D) TOPOLOGY: linear

       (ii) MOLECULE TYPE: protein

       (xi) SEQUENCE DESCRIPTION: SEQ ID NO:2:

Met Gly Ala Phe Thr Phe Lys His Ser Phe Phe Gly Ser Phe Val Glu
  1               5                  10                  15

Cys Ser Gly Val Leu Gln Thr Val Phe Ile Phe Leu Leu Ile Pro Cys
             20                  25                  30

Cys Leu Ala Asp Lys Arg Ala Pro Pro Leu Ile Pro Asn Val Pro Leu
         35                  40                  45

Leu Trp Val Trp Asn Ala Pro Thr Glu Phe Cys Ile Gly Gly Thr Asn
     50                  55                  60

Gln Pro Leu Asp Met Ser Phe Phe Ser Ile Val Gly Thr Pro Arg Lys
 65                  70                  75                  80

Asn Ile Thr Gly Gln Ser Ile Thr Leu Tyr Tyr Val Asp Arg Leu Gly
                 85                  90                  95

Tyr Tyr Pro Tyr Ile Asp Pro His Thr Gly Ala Ile Val His Gly Gly
            100                 105                 110

Leu Pro Gln Leu Met Asn Leu Gln Gln His Leu Arg Lys Ser Arg Gln
        115                 120                 125

Asp Ile Leu Phe Tyr Met Pro Thr Asp Ser Val Gly Leu Ala Val Ile
    130                 135                 140

Asp Trp Glu Glu Trp Arg Pro Thr Trp Thr Arg Asn Trp Arg Pro Lys
145                 150                 155                 160

Asp Ile Tyr Arg Asn Lys Ser Ile Glu Leu Val Lys Ser Gln His Pro
                165                 170                 175

Gln Tyr Asn His Ser Tyr Ala Val Ala Val Ala Lys Arg Asp Phe Glu
            180                 185                 190

Arg Thr Gly Lys Ala Phe Met Leu Glu Thr Leu Lys Leu Gly Lys Ser
        195                 200                 205

Leu Arg Pro Ser Ser Leu Trp Gly Tyr Tyr Leu Phe Pro Asp Cys Tyr
    210                 215                 220

Asn Thr His Phe Thr Lys Pro Asn Tyr Asp Gly His Cys Pro Pro Ile
225                 230                 235                 240

Glu Leu Gln Arg Asn Asn Asp Leu Gln Trp Leu Trp Asn Asp Ser Thr
                245                 250                 255

Ala Leu Tyr Pro Ser Val Tyr Leu Thr Ser Arg Val Arg Ser Ser Gln
            260                 265                 270

Asn Gly Ala Leu Tyr Val Arg Asn Arg Val His Glu Ser Ile Arg Val
        275                 280                 285

Ser Lys Leu Met Asp Asp Lys Asn Pro Leu Pro Ile Tyr Val Tyr Ile
    290                 295                 300

Arg Leu Val Phe Thr Asp Gln Thr Thr Thr Phe Leu Glu Leu Asp Asp
305                 310                 315                 320

Leu Val His Ser Val Gly Glu Ile Val Pro Leu Gly Val Ser Gly Ile
                325                 330                 335

Ile Ile Trp Gly Ser Leu Ser Leu Thr Arg Ser Leu Val Ser Cys Ile
            340                 345                 350

Gly Leu Glu Asn Tyr Met Lys Gly Thr Leu Leu Pro Tyr Leu Ile Asn
        355                 360                 365

Val Thr Leu Ala Ala Lys Met Cys Gly Gln Val Leu Cys Lys Asn Gln
    370                 375                 380

Gly Ile Cys Thr Arg Lys Asp Trp Asn Thr Asn Thr Tyr Leu His Leu
385                 390                 395                 400

Asn Ala Thr Asn Phe Asp Ile Glu Leu Gln Gln Asn Gly Lys Phe Val
                405                 410                 415

Val His Gly Lys Pro Ser Leu Glu Asp Leu Gln Glu Phe Ser Lys Asn
            420                 425                 430

Phe His Cys Ser Cys Tyr Thr Asn Val Ala Cys Lys Asp Arg Leu Asp
        435                 440                 445

Val His Asn Val Arg Ser Val Asn Val Cys Thr Ala Asn Asn Ile Cys
    450                 455                 460

Ile Asp Ala Val Leu Asn Phe Pro Ser Leu Asp Asp Asp Asp Glu Pro
465                 470                 475                 480

Pro Ile Thr Asp Asp Thr Ser Gln Asn Gln Asp Ser Ile Ser Asp Ile
                485                 490                 495

Thr Ser Ser Ala Pro Pro Ser Ser His Ile Leu Pro Lys Asp Leu Ser
            500                 505                 510

Trp Cys Leu Phe Leu Leu Ser Ile Phe Ser Gln His Trp Lys Tyr Leu
        515                 520                 525

Leu

(2) INFORMATION FOR SEQ ID NO:3:

(i) SEQUENCE CHARACTERISTICS:
(A) LENGTH: 2125 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: CDS
(B) LOCATION: 313..1848

(xi) SEQUENCE DESCRIPTION: SEQ ID NO:3:

CTAAGAGTGC TGAAGTAGAT TTAGATTGAC CATGGCTCAC ATGAATTAAG AAGTGTTTTC 60

TTTTGTTATG ATGGAGATGC GAGTGGTAGG CAGGTATTTT AAGTTTCCAG CAAGTTCTGG 120

ATGATTTAAC TTGCTCCAAG ATATTCCTGA AATGTAACAC AGGAAGAAGA ATCTTCAGTG 180

TAAATCAGTC ACCATACATT CATCTCCCTC AATAGCCTCA TGCCACAGTC TTTCTAATCT 240

TTTGCATCTA ATACTAAACA GACCACAGTG TGTAAGAAGG AATAAGTGCC TACTTAGTAA 300

TTATTCTCTG TG ATG GGA GAG TTG AGA TTT AAG CAC CTC TTT TGG GGG 348
Met Gly Glu Leu Arg Phe Lys His Leu Phe Trp Gly
1               5                   10

AGC TTT GTT GAA CTC GGG GGC ACA TTC CAA ACA GTG TTA ATC TTC CTT 396
Ser Phe Val Glu Leu Gly Gly Thr Phe Gln Thr Val Leu Ile Phe Leu
        15                  20                  25

TTG ATT CCA TGC TCC TTG ACT GTG GAT TAT AGG GCA GCA CCA ATT TTA 444
Leu Ile Pro Cys Ser Leu Thr Val Asp Tyr Arg Ala Ala Pro Ile Leu
    30                  35                  40

TCA AAT ACA ACT TTC CTT TGG ATT TGG AAT GTC CCA ACT GAA CGT TGT 492
Ser Asn Thr Thr Phe Leu Trp Ile Trp Asn Val Pro Thr Glu Arg Cys
45                  50                  55                  60

GTA GGA AAT [?][?]T AAT GAT CCA ATA GAT CTG AGC TTC TTC TCT TTA ATT 540
Val Gly Asr [?][?][?] l Asn Asp Pro Ile Asp Leu Ser Phe Phe Ser Leu Ile
65 70 75

GGA AGC CCC [?]GG AAA ACT GCC ACA GGG CAA CCT GTC ACA TTA TTT TAT 588
Gly Ser Pro Arg Lys Thr Ala Thr Gly Gln Pro Val Thr Leu Phe Tyr
80 85 90

GTT GAT CGA CTT GGT TTG TAT CCT CAC ATA GAT GCA AAC CAA GCA GAA 636
Val Asp Arg Leu Gly Leu Tyr Pro His Ile Asp Ala Asn Gln Ala Glu
95 100 105

CAT TAT GGA GGA ATA CCT CAG AGG GGC GAT TAT CAA GCT CAT TTG CGC 684
His Tyr Gly Gly Ile Pro Gln Arg Gly Asp Tyr Gln Ala His Leu Arg
110 115 120

AAA GCT AAG ACT GAC ATA GAG CAT TAC ATT CCA GAC GAC AAA TTG GGC 732
Lys Ala Lys Thr Asp Ile Glu His Tyr Ile Pro Asp Asp Lys Leu Gly
125 130 135 140

TTA GCT ATC ATT GAC TGG GAA GAA TGG AGG CCT ACC TGG TTG AGA AAC 780
Leu Ala Ile Ile Asp Trp Glu Glu Trp Arg Pro Thr Trp Leu Arg Asn
145 150 155

TGG AAA CCT AAG GAT AAC TAC AGG AAT AAG TCT ATT GAA TTG GTC CAA 828
Trp Lys Pro Lys Asp Asn Tyr Arg Asn Lys Ser Ile Glu Leu Val Gln
160 165 170

TCA ACT AAT CCA GGA CTT AGT ATC ACA AGA GCC ACC CAG AAA GCC ATA 876
Ser Thr Asn Pro Gly Leu Ser Ile Thr Arg Ala Thr Gln Lys Ala Ile
175 180 185

CAA CAA CTT GAA GAG GCA GGA AGG AAG TTT ATG GAA GGA ACT TTA CAC 924
Gln Gln Leu Glu Glu Ala Gly Arg Lys Phe Met Glu Gly Thr Leu His
190 195 200

CTG GGG AAA TTC CTT CGA CCA AAC CAG CTA TGG GGT TAT TAT CTA TTT 972
Leu Gly Lys Phe Leu Arg Pro Asn Gln Leu Trp Gly Tyr Tyr Leu Phe
205 210 215 220

CCT GAT TGT TAT AAC AAT AAG TTT CAA GAC CCT AAG TAT GAT GGG CAG 1020
Pro Asp Cys Tyr Asn Asn Lys Phe Gln Asp Pro Lys Tyr Asp Gly Gln
225 230 235

TGC CCT GCT GTG GAA AAG AAA AGA AAT GAT AAT CTT AAA TGG CTA TGG 1068
Cys Pro Ala Val Glu Lys Lys Arg Asn Asp Asn Leu Lys Trp Leu Trp
240 245 250

AAA GCA AGC ACC GGC CTT TAC CCA TCT GTC TAT TTG AAG AAA GAC TTG 1116
Lys Ala Ser Thr Gly Leu Tyr Pro Ser Val Tyr Leu Lys Lys Asp Leu
255 260 265

AAG TCC AAT CGA CAA GCT ACC CTC TAT GTC CGC TAC CGA GTT GTG GAA 1164
Lys Ser Asn Arg Gln Ala Thr Leu Tyr Val Arg Tyr Arg Val Val Glu
270 275 280

GCT ATC AGA GTG TCC AAG GTT GGG AAT GCA TCG GAT CCA GTC CCG ATT 1212
Ala Ile Arg Val Ser Lys Val Gly Asn Ala Ser Asp Pro Val Pro Ile
285 290 295 300

TTT GTC TAT ATC CGT CTT GTT TTT ACT GAT CGT ACC TCT GAA TAC CTT 1260
Phe Val Tyr Ile Arg Leu Val Phe Thr Asp Arg Thr Ser Glu Tyr Leu
305 310 315

CTA GAG GAT GAC CTT GTG AAT ACA ATT GGT GAA ATT GTT GCT CTG GGT 1308
Leu Glu Asp Asp Leu Val Asn Thr Ile Gly Glu Ile Val Ala Leu Gly
320 325 330

ACC TCT GGA ATT ATA ATA TGG GAT GCT ATG AGT TTA GCA CAA CGT GCG 1356
Thr Ser Gly Ile Ile Ile Trp Asp Ala Met Ser Leu Ala Gln Arg Ala
335 340 345

GCA GGT TGC CCA ATC CTA CAT AAA TAC ATG CAG ACG ACC CTG AAT CCA 1404
Ala Gly Cys Pro Ile Leu His Lys Tyr Met Gln Thr Thr Leu Asn Pro
350 355 360

TAC ATA GTC AAT GTT ACC CTA GCA GCC AAA ATG TGC AGC CAA ACA CTT 1452
Tyr Ile Val Asn Val Thr Leu Ala Ala Lys Met Cys Ser Gln Thr Leu
365 370 375 380

TGT AAT GAG AAA GGC ATG TGT TCA AGA AGA AAA GAA AGT TCA GAT GTA 1500
Cys Asn Glu Lys Gly Met Cys Ser Arg Arg Lys Glu Ser Ser Asp Val
385 390 395

TAT CTT CAC TTG AAC CCA AGT CAT TTT GAT ATT ATG TTA ACG CAA ACT 1548
Tyr Leu His Leu Asn Pro Ser His Phe Asp Ile Met Leu Thr Gln Thr
400 405 410

GGA AAG TAC GAA GTT CTT GGC AAC CCC AGG GTT GGA GAC TTA GAA TAC 1596
Gly Lys Tyr Glu Val Leu Gly Asn Pro Arg Val Gly Asp Leu Glu Tyr
415 420 425

TTT TCT GAA CAT TTT AAA TGC AGC TGT TTT AGC AGA ATG ACA TGT AAG 1644
Phe Ser Glu His Phe Lys Cys Ser Cys Phe Ser Arg Met Thr Cys Lys
430 435 440

GAG ACA TCT GAT GTA AAA AAT GTA CAA GAC GTG AAT GTG TGC GTC GGT 1692
Glu Thr Ser Asp Val Lys Asn Val Gln Asp Val Asn Val Cys Val Gly
445 450 455 460

GAC AAT GTT TGT ATA AAA GCC AAG GTA GAA CCC AAC CCA GCC TTC TAC 1740
Asp Asn Val Cys Ile Lys Ala Lys Val Glu Pro Asn Pro Ala Phe Tyr
465 470 475

CTC CTA CCT GGC AAA AGC CTT CTA TTT ATG ACA ACA CTT GGT CAT GTG 1788
Leu Leu Pro Gly Lys Ser Leu Leu Phe Met Thr Thr Leu Gly His Val
480 485 490

CTG TAC CAT CTG CCA CAA GAT ATT TTT GTT TTT CCA CGG AAG ACA CTA 1836
Leu Tyr His Leu Pro Gln Asp Ile Phe Val Phe Pro Arg Lys Thr Leu
495 500 505

GTC AGT ACT CCT TAGTTTTCTC TACCCACAGC GTTTGATGTA TTATTATTAT 1888
Val Ser Thr Pro
510

TATTTTTGCA GGCCTCAGTA ATTTGGATTT ATGAATGGGA TTCTATTTTA CCAAAGTAAT 1948

TCAATTTTTA TAATCAAGAT TCTATTTTTG AGTTTCAAAG AGAAATTATA TATTCTTCTA 2008

CCAAAGATTG ATTACAAGCA AGGCTACTTA GGGATTAGTT TTGGTTTAAA GAGAATGAAG 2068

ACTGAATAAA ATAAAATCAC TAGAAAATTA AAAAAAAAAA AAAAAAAAAA AAAAAAA 2125

(2) INFORMATION FOR SEQ ID NO:4:

(i) SEQUENCE CHARACTERISTICS:
(A) LENGTH: 512 amino acids
(B) TYPE: amino acid
(D) TOPOLOGY: linear

(ii) MOLECULE TYPE: protein

(xi) SEQUENCE DESCRIPTION: SEQ ID NO:4:

Met Gly Glu Leu Arg Phe Lys His Leu Phe Trp Gly Ser Phe Val Glu
1               5                   10                  15

Leu Gly Gly Thr Phe Gln Thr Val Leu Ile Phe Leu Leu Ile Pro Cys
            20                  25                  30

Ser Leu Thr Val Asp Tyr Arg Ala Ala Pro Ile Leu Ser Asn Thr Thr
        35                  40                  45

Phe Leu Trp Ile Trp Asn Val Pro Thr Glu Arg Cys Val Gly Asn Val
    50                  55                  60

Asn Asp Pro Ile Asp Leu Ser Phe Phe Ser Leu Ile Gly Ser Pro Arg
65                  70                  75                  80

Lys Thr Ala Thr Gly Gln Pro Val Thr Leu Phe Tyr Val Asp Arg Leu
                85                  90                  95

Gly Leu Tyr Pro His Ile Asp Ala Asn Gln Ala Glu His Tyr Gly Gly
            100                 105                 110

Ile Pro Gln Arg Gly Asp Tyr Gln Ala His Leu Arg Lys Ala Lys Thr
        115                 120                 125

Asp Ile Glu His Tyr Ile Pro Asp Asp Lys Leu Gly Leu Ala Ile Ile
    130                 135                 140

Asp Trp Glu Glu Trp Arg Pro Thr Trp Leu Arg Asn Trp Lys Pro Lys
145                 150                 155                 160

Asp Asn Tyr Arg Asn Lys Ser Ile Glu Leu Val Gln Ser Thr Asn Pro
                165                 170                 175

Gly Leu Ser Ile Thr Arg Ala Thr Gln Lys Ala Ile Gln Gln Leu Glu
            180                 185                 190

Glu Ala Gly Arg Lys Phe Met Glu Gly Thr Leu His Leu Gly Lys Phe
        195                 200                 205

Leu Arg Pro Asn Gln Leu Trp Gly Tyr Tyr Leu Phe Pro Asp Cys Tyr
    210                 215                 220

Asn Asn Lys Phe Gln Asp Pro Lys Tyr Asp Gly Gln Cys Pro Ala Val
225                 230                 235                 240

Glu Lys Lys Arg Asn Asp Asn Leu Lys Trp Leu Trp Lys Ala Ser Thr
                245                 250                 255

Gly Leu Tyr Pro Ser Val Tyr Leu Lys Lys Asp Leu Lys Ser Asn Arg
            260                 265                 270

Gln Ala Thr Leu Tyr Val Arg Tyr Arg Val Val Glu Ala Ile Arg Val
        275                 280                 285

Ser Lys Val Gly Asn Ala Ser Asp Pro Val Pro Ile Phe Val Tyr Ile
    290                 295                 300

Arg Leu Val Phe Thr Asp Arg Thr Ser Glu Tyr Leu Leu Glu Asp Asp
305                 310                 315                 320

Leu Val Asn Thr Ile Gly Glu Ile Val Ala Leu Gly Thr Ser Gly Ile
                325                 330                 335

Ile Ile Trp Asp Ala Met Ser Leu Ala Gln Arg Ala Ala Gly Cys Pro
            340                 345                 350

Ile Leu His Lys Tyr Met Gln Thr Thr Leu Asn Pro Tyr Ile Val Asn
        355                 360                 365

Val Thr Leu Ala Ala Lys Met Cys Ser Gln Thr Leu Cys Asn Glu Lys
    370                 375                 380

Gly Met Cys Ser Arg Arg Lys Glu Ser Ser Asp Val Tyr Leu His Leu
385                 390                 395                 400

Asn Pro Ser His Phe Asp Ile Met Leu Thr Gln Thr Gly Lys Tyr Glu
                405                 410                 415

Val Leu Gly Asn Pro Arg Val Gly Asp Leu Glu Tyr Phe Ser Glu His
            420                 425                 430

Phe Lys Cys Ser Cys Phe Ser Arg Met Thr Cys Lys Glu Thr Ser Asp
        435                 440                 445

Val Lys Asn Val Gln Asp Val Asn Val Cys Val Gly Asp Asn Val Cys
    450                 455                 460

Ile Lys Ala Lys Val Glu Pro Asn Pro Ala Phe Tyr Leu Leu Pro Gly
465                 470                 475                 480

Lys Ser Leu Leu Phe Met Thr Thr Leu Gly His Val Leu Tyr His Leu
                485                 490                 495

Pro Gln Asp Ile Phe Val Phe Pro Arg Lys Thr Leu Val Ser Thr Pro
            500                 505                 510

(2) INFORMATION FOR SEQ ID NO:5:

     (i) SEQUENCE CHARACTERISTICS:
          (A) LENGTH: 1695 base pairs
          (B) TYPE: nucleic acid
          (C) STRANDEDNESS: double
          (D) TOPOLOGY: linear

    (ii) MOLECULE TYPE: DNA (genomic)

    (ix) FEATURE:
          (A) NAME/KEY: CDS
          (B) LOCATION: 109..1635

(xi) SEQUENCE DESCRIPTION: SEQ ID NO:5:

GGAATTCATT CCATTCCCTT TCATCTGTGC TCATACTTTG CATCAGATAT TGGGTAAACC 60

AAAGTGTGTA GGAAGAAATA AATGTTTTCA TAGTCATTAC TCTTTACA ATG GGA GTG 117
Met Gly Val
1

CTA AAA TTC AAG CAC ATC TTT TTC AGA AGC TTT GTT AAA TCA AGT GGA 165
Leu Lys Phe Lys His Ile Phe Phe Arg Ser Phe Val Lys Ser Ser Gly
    5                   10                  15

GTA TCC CAG ATA GTT TTC ACC TTC CTT CTG ATT CCA TGT TGC TTG ACT 213
Val Ser Gln Ile Val Phe Thr Phe Leu Leu Ile Pro Cys Cys Leu Thr
20                  25                  30                  35

CTG AAT TTC AGA GCA CCT CCT GTT ATT CCA AAT GTG CCT TTC CTC TGG 261
Leu Asn Phe Arg Ala Pro Pro Val Ile Pro Asn Val Pro Phe Leu Trp
                40                  45                  50

GCC TGG AAT GCC CCA AGT GAA TTT TGT CTT GGA AAA TTT GAT GAG CCA 309
Ala Trp Asn Ala Pro Ser Glu Phe Cys Leu Gly Lys Phe Asp Glu Pro
            55                  60                  65

CTA GAT ATG AGC CTC TTC TCT TTC ATA GGA AGC CCC CGA ATA AAC GCC 357
Leu Asp Met Ser Leu Phe Ser Phe Ile Gly Ser Pro Arg Ile Asn Ala
        70                  75                  80

ACC GGG CAA GGA GTT ACA ATA TTT TAT GTT GAT AGA CTT GGC TAC TAT 405
Thr Gly Gln Gly Val Thr Ile Phe Tyr Val Asp Arg Leu Gly Tyr Tyr
    85                  90                  95

CCT TAC ATA GAT TCA ATC ACA GGA GTA ACT GTG AAT GGA GGA ATC CCC 453
Pro Tyr Ile Asp Ser Ile Thr Gly Val Thr Val Asn Gly Gly Ile Pro
100                 105                 110                 115

CAG AAG ATT TCC TTA CAA GAC CAT CTG GAC AAA GCT AAG AAA GAC ATT 501
Gln Lys Ile Ser Leu Gln Asp His Leu Asp Lys Ala Lys Lys Asp Ile
                120                 125                 130

ACA TTT TAT ATG CCA GTA GAC AAT TTG GGA ATG GCT GTT ATT GAC TGG 549
Thr Phe Tyr Met Pro Val Asp Asn Leu Gly Met Ala Val Ile Asp Trp
            135                 140                 145

GAA GAA TGG AGA CCC ACT TGG GCA AGA AAC TGG AAA CCT AAA GAT GTT 597
Glu Glu Trp Arg Pro Thr Trp Ala Arg Asn Trp Lys Pro Lys Asp Val
        150                 155                 160

TAC AAG AAT AGG TCT ATT GAA TTG GTT CAG CAA CAA AAT GTA CAA CTT 645
Tyr Lys Asn Arg Ser Ile Glu Leu Val Gln Gln Gln Asn Val Gln Leu
    165                 170                 175

AGT CTC ACA GAG GCC ACT GAG AAA GCA AAA CAA GAA TTT GAA AAG GCA 693
Ser Leu Thr Glu Ala Thr Glu Lys Ala Lys Gln Glu Phe Glu Lys Ala
180                 185                 190                 195

GGG AAG GAT TTC CTG GTA GAG ACT ATA AAA TTG GGA AAA TTA CTT CGG 741
Gly Lys Asp Phe Leu Val Glu Thr Ile Lys Leu Gly Lys Leu Leu Arg
                200                 205                 210

CCA AAT CAC TTG TGG GGT TAT TAT CTT TTT CCG GAT TGT TAC AAC CAT 789
Pro Asn His Leu Trp Gly Tyr Tyr Leu Phe Pro Asp Cys Tyr Asn His
            215                 220                 225

CAC TAT AAG AAA CCC GGT TAC AAT GGA AGT TGC TTC AAT GTA GAA ATA 837
His Tyr Lys Lys Pro Gly Tyr Asn Gly Ser Cys Phe Asn Val Glu Ile
        230                 235                 240

AAA AGA AAT GAT GAT CTC AGC TGG TTG TGG AAT GAA AGC ACT GCT CTT 885
Lys Arg Asn Asp Asp Leu Ser Trp Leu Trp Asn Glu Ser Thr Ala Leu
    245                 250                 255

TAC CCA TCC ATT TAT TTG AAC ACT CAG CAG TCT CCT GTA GCT GCT ACA 933
Tyr Pro Ser Ile Tyr Leu Asn Thr Gln Gln Ser Pro Val Ala Ala Thr
260                 265                 270                 275

CTC TAT GTG CGC AAT CGA GTT CGG GAA GCC ATC AGA GTT TCC AAA ATA 981
Leu Tyr Val Arg Asn Arg Val Arg Glu Ala Ile Arg Val Ser Lys Ile
                280                 285                 290

CCT GAT GCA AAA AGT CCA CTT CCG GTT TTT GCA TAT ACC CGC ATA GTT 1029
Pro Asp Ala Lys Ser Pro Leu Pro Val Phe Ala Tyr Thr Arg Ile Val
            295                 300                 305

TTT ACT GAT CAA GTT TTG AAA TTC CTT TCT CAA GAT GAA CTT GTG TAT 1077
Phe Thr Asp Gln Val Leu Lys Phe Leu Ser Gln Asp Glu Leu Val Tyr
        310                 315                 320

ACA TTT GGC GAA ACT GTT GCT CTG GGT GCT TCT GGA ATT GTA ATA TGG 1125
Thr Phe Gly Glu Thr Val Ala Leu Gly Ala Ser Gly Ile Val Ile Trp
    325                 330                 335

GGA ACC CTC AGT ATA ATG CGA AGT ATG AAA TCT TGC TTG CTC CTA GAC 1173
Gly Thr Leu Ser Ile Met Arg Ser Met Lys Ser Cys Leu Leu Leu Asp
340                 345                 350                 355

AAT TAC ATG GAG ACT ATA CTG AAT CCT TAC ATA ATC AAC GTC ACA CTA 1221
Asn Tyr Met Glu Thr Ile Leu Asn Pro Tyr Ile Ile Asn Val Thr Leu
                360                 365                 370

GCA GCC AAA ATG TGT AGC CAA GTG CTT TGC CAG GAG CAA GGA GTG TGT 1269
Ala Ala Lys Met Cys Ser Gln Val Leu Cys Gln Glu Gln Gly Val Cys
            375                 380                 385

ATA AGG AAA AAC TGG AAT TCA AGT GAC TAT CTT CAC CTC AAC CCA GAT 1317
Ile Arg Lys Asn Trp Asn Ser Ser Asp Tyr Leu His Leu Asn Pro Asp
        390                 395                 400

AAT TTT GCT ATT CAA CTT GAG AAA GGT GGA AAG TTC ACA GTA CGT GGA 1365
Asn Phe Ala Ile Gln Leu Glu Lys Gly Gly Lys Phe Thr Val Arg Gly
    405                 410                 415

AAA CCG ACA CTT GAA GAC CTG GAG CAA TTT TCT GAA AAA TTT TAT TGC 1413
Lys Pro Thr Leu Glu Asp Leu Glu Gln Phe Ser Glu Lys Phe Tyr Cys
420                 425                 430                 435

AGC TGT TAT AGC ACC TTG AGT TGT AAG GAG AAA GCT GAT GTA AAA GAC 1461
Ser Cys Tyr Ser Thr Leu Ser Cys Lys Glu Lys Ala Asp Val Lys Asp
                440                 445                 450

ACT GAT GCT GTT GAT GTG TGT ATT GCT GAT GGT GTC TGT ATA GAT GCT 1509
Thr Asp Ala Val Asp Val Cys Ile Ala Asp Gly Val Cys Ile Asp Ala
            455                 460                 465

TTT CTA AAA CCT CCC ATG GAG ACA GAA GAA CCT CAA ATT TTC TAC AAT 1557
Phe Leu Lys Pro Pro Met Glu Thr Glu Glu Pro Gln Ile Phe Tyr Asn
        470                 475                 480

GCT TCA CCC TCC ACA CTA TCT GCC ACA ATG TTC ATT GTT AGT ATT TTG 1605
Ala Ser Pro Ser Thr Leu Ser Ala Thr Met Phe Ile Val Ser Ile Leu
    485                 490                 495

TTT CTT ATC ATT TCT TCT GTA GCG AGT TTG TAATTGCGCA GGTTAGCTGA 1655
Phe Leu Ile Ile Ser Ser Val Ala Ser Leu
500                 505

AATGAACAAT ATGTCCATCT TAAAGTGTGC TTCCCGAATT 1695

(2) INFORMATION FOR SEQ ID NO:6:

(i) SEQUENCE CHARACTERISTICS:
(A) LENGTH: 509 amino acids
(B) TYPE: amino acid
(D) TOPOLOGY: linear

(ii) MOLECULE TYPE: protein

(xi) SEQUENCE DESCRIPTION: SEQ ID NO:6:

Met Gly Val Leu Lys Phe Lys His Ile Phe Phe Arg Ser Phe Val Lys
1               5                   10                  15

Ser Ser Gly Val Ser Gln Ile Val Phe Thr Phe Leu Leu Ile Pro Cys
            20                  25                  30

Cys Leu Thr Leu Asn Phe Arg Ala Pro Pro Val Ile Pro Asn Val Pro
        35                  40                  45

Phe Leu Trp Ala Trp Asn Ala Pro Ser Glu Phe Cys Leu Gly Lys Phe
    50                  55                  60

Asp Glu Pro Leu Asp Met Ser Leu Phe Ser Phe Ile Gly Ser Pro Arg
65                  70                  75                  80

Ile Asn Ala Thr Gly Gln Gly Val Thr Ile Phe Tyr Val Asp Arg Leu
                85                  90                  95

Gly Tyr Tyr Pro Tyr Ile Asp Ser Ile Thr Gly Val Thr Val Asn Gly
            100                 105                 110

Gly Ile Pro Gln Lys Ile Ser Leu Gln Asp His Leu Asp Lys Ala Lys
        115                 120                 125

Lys Asp Ile Thr Phe Tyr Met Pro Val Asp Asn Leu Gly Met Ala Val
    130                 135                 140

Ile Asp Trp Glu Glu Trp Arg Pro Thr Trp Ala Arg Asn Trp Lys Pro
145                 150                 155                 160

Lys Asp Val Tyr Lys Asn Arg Ser Ile Glu Leu Val Gln Gln Gln Asn
                165                 170                 175

Val Gln Leu Ser Leu Thr Glu Ala Thr Glu Lys Ala Lys Gln Glu Phe
            180                 185                 190

Glu Lys Ala Gly Lys Asp Phe Leu Val Glu Thr Ile Lys Leu Gly Lys
        195                 200                 205

Leu Leu Arg Pro Asn His Leu Trp Gly Tyr Tyr Leu Phe Pro Asp Cys
    210                 215                 220

Tyr Asn His His Tyr Lys Lys Pro Gly Tyr Asn Gly Ser Cys Phe Asn
225                 230                 235                 240

Val Glu Ile Lys Arg Asn Asp Asp Leu Ser Trp Leu Trp Asn Glu Ser
                245                 250                 255

Thr Ala Leu Tyr Pro Ser Ile Tyr Leu Asn Thr Gln Gln Ser Pro Val
            260                 265                 270

Ala Ala Thr Leu Tyr Val Arg Asn Arg Val Arg Glu Ala Ile Arg Val
        275                 280                 285

Ser Lys Ile Pro Asp Ala Lys Ser Pro Leu Pro Val Phe Ala Tyr Thr
    290                 295                 300

Arg Ile Val Phe Thr Asp Gln Val Leu Lys Phe Leu Ser Gln Asp Glu
305                 310                 315                 320

Leu Val Tyr Thr Phe Gly Glu Thr Val Ala Leu Gly Ala Ser Gly Ile
                325                 330                 335

Val Ile Trp Gly Thr Leu Ser Ile Met Arg Ser Met Lys Ser Cys Leu
            340                 345                 350

Leu Leu Asp Asn Tyr Met Glu Thr Ile Leu Asn Pro Tyr Ile Ile Asn
        355                 360                 365

Val Thr Leu Ala Ala Lys Met Cys Ser Gln Val Leu Cys Gln Glu Gln
    370                 375                 380

Gly Val Cys Ile Arg Lys Asn Trp Asn Ser Ser Asp Tyr Leu His Leu
385                 390                 395                 400

Asn Pro Asp Asn Phe Ala Ile Gln Leu Glu Lys Gly Gly Lys Phe Thr
                405                 410                 415

Val Arg Gly Lys Pro Thr Leu Glu Asp Leu Glu Gln Phe Ser Glu Lys
            420                 425                 430

Phe Tyr Cys Ser Cys Tyr Ser Thr Leu Ser Cys Lys Glu Lys Ala Asp
        435                 440                 445

Val Lys Asp Thr Asp Ala Val Asp Val Cys Ile Ala Asp Gly Val Cys
    450                 455                 460

Ile Asp Ala Phe Leu Lys Pro Pro Met Glu Thr Glu Glu Pro Gln Ile
465                 470                 475                 480

Phe Tyr Asn Ala Ser Pro Ser Thr Leu Ser Ala Thr Met Phe Ile Val
                485                 490                 495

Ser Ile Leu Phe Leu Ile Ile Ser Ser Val Ala Ser Leu
            500                 505

(2) INFORMATION FOR SEQ ID NO:7:

(i) SEQUENCE CHARACTERISTICS:
(A) LENGTH: 1674 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: CDS
(B) LOCATION: 1..825

(xi) SEQUENCE DESCRIPTION: SEQ ID NO:7:

GGA AGT TGC TTC AAT GTA GAA ATA AAA AGA AAT GAT GAT CTC AGC TGG 48
Gly Ser Cys Phe Asn Val Glu Ile Lys Arg Asn Asp Asp Leu Ser Trp
1               5                   10                  15

TTG TGG AAT GAA AGC ACT GCT CTT TAC CCA TCC ATT TAT TTG AAC ACT 96
Leu Trp Asn Glu Ser Thr Ala Leu Tyr Pro Ser Ile Tyr Leu Asn Thr
            20                  25                  30

CAG CAG TCT CCT GTA GCT GCT ACA CTC TAT GTG CGC AAT CGA GTT CGG 144
Gln Gln Ser Pro Val Ala Ala Thr Leu Tyr Val Arg Asn Arg Val Arg
        35                  40                  45

GAA GCC ATC AGA GTT TCC AAA ATA CCT GAT GCA AAA AGT CCA CTT CCG 192
Glu Ala Ile Arg Val Ser Lys Ile Pro Asp Ala Lys Ser Pro Leu Pro
    50                  55                  60

GTT TTT GCA TAT ACC CGC ATA GTT TTT ACT GAT CAA GTT TTG AAA TTC 240
Val Phe Ala Tyr Thr Arg Ile Val Phe Thr Asp Gln Val Leu Lys Phe
65                  70                  75                  80

CTT TCT CAA GAT GAA CTT GTG TAT ACA TTT GGC GAA ACT GTT GCT CTG 288
Leu Ser Gln Asp Glu Leu Val Tyr Thr Phe Gly Glu Thr Val Ala Leu
                85                  90                  95

GGT GCT TCT GGA ATT GTA ATA TGG GGA ACC CTC AGT ATA ATG CGA AGT 336
Gly Ala Ser Gly Ile Val Ile Trp Gly Thr Leu Ser Ile Met Arg Ser
            100                 105                 110

ATG AAA TCT TGC TTG CTC CTA GAC AAT TAC ATG GAG ACT ATA CTG AAT 384
Met Lys Ser Cys Leu Leu Leu Asp Asn Tyr Met Glu Thr Ile Leu Asn
        115                 120                 125

CCT TAC ATA ATC AAC GTC ACA CTA GCA GCC AAA ATG TGT AGC CAA GTG 432
Pro Tyr Ile Ile Asn Val Thr Leu Ala Ala Lys Met Cys Ser Gln Val
    130                 135                 140

CTT TGC CAG GAG CAA GGA GTG TGT ATA AGG AAA AAC TGG AAT TCA AGT 480
Leu Cys Gln Glu Gln Gly Val Cys Ile Arg Lys Asn Trp Asn Ser Ser
145                 150                 155                 160

GAC TAT CTT CAC CTC AAC CCA GAT AAT TTT GCT ATT CAA CTT GAG AAA 528
Asp Tyr Leu His Leu Asn Pro Asp Asn Phe Ala Ile Gln Leu Glu Lys
                165                 170                 175

GGT GGA AAG TTC ACA GTA CGT GGA AAA CCG ACA CTT GAA GAC CTG GAG 576
Gly Gly Lys Phe Thr Val Arg Gly Lys Pro Thr Leu Glu Asp Leu Glu
            180                 185                 190

CAA TTT TCT GAA AAA TTT TAT TGC AGC TGT TAT AGC ACC TTG AGT TGT 624
Gln Phe Ser Glu Lys Phe Tyr Cys Ser Cys Tyr Ser Thr Leu Ser Cys
        195                 200                 205

AAG GAG AAA GCT GAT GTA AAA GAC ACT GAT GCT GTT GAT GTG TGT ATT 672
Lys Glu Lys Ala Asp Val Lys Asp Thr Asp Ala Val Asp Val Cys Ile
    210                 215                 220

GCT GAT GGT GTC TGT ATA GAT GCT TTT CTA AAA CCT CCC ATG GAG ACA 720
Ala Asp Gly Val Cys Ile Asp Ala Phe Leu Lys Pro Pro Met Glu Thr
225                 230                 235                 240

GAA GAA CCT CAA ATT TTC TAC AAT GCT TCA CCC TCC ACA CTA TCT GCC 768
Glu Glu Pro Gln Ile Phe Tyr Asn Ala Ser Pro Ser Thr Leu Ser Ala
                245                 250                 255

ACA ATG TTC ATT GAT CTT TGT GAC CTG TAT CTT GTG CCA ACC TCC TAT 816
Thr Met Phe Ile Asp Leu Cys Asp Leu Tyr Leu Val Pro Thr Ser Tyr
            260                 265                 270

CTC ATC CTG TGACTAAGAA TACTTAACCT CCTGGGAGGG CAGCTCAGGA 865
Leu Ile Leu
        275

GGTGTCAGCC TCATTTTACC CAGCCCTATT CAAGATGGAG TCACTCTGGT TCCAATGCTT 925

CTGACAGCAG TAGAGATAAC ACAGTATTCA AGCAAGAGAA CAGAGCTCCT GATCACCTGT 985

GTGCGTCCTT TGAGTGGATG GCAGCTGCAT CTCTGCATTA CAGCTAGTTA GAATGATGAG 1045

TCCTTGCTAT GCCTCAAGCA CTGTTTCGAG TGTTTGATGT CTATTATCTC ACTTCATCCT 1105

CACCAGGACC CCATCCGAGC CTTAATTTCA GTTGACAGTA ACTATTGGAT CCCCAGGAAT 1165

ATGTTTGCAT ATTTGGGGAG AAAATACTAT TGGAGGGGAA CAGAAATGCT ACTAAGGGTC 1225

TCACTGTGTC ACCCAGGCTG GAGTCCATCA AAGCTCACTG CAGCCTTAAC CTTCTGTGCT 1285

CAAGGGATCC TCCCACTTAA GCCTCCTGAG TAGCTGGAAC TACAGGCATA TGCCACCGAG 1345

CCTGGCTAAT CTTTGATTTT TTTGTACAGA TTGTGTCTCC TTATGTTGCT CAGGCTGGAC 1405

TCAAACTTCT GGTCTCAAGC GATCTTTCCA TCTTAGCTTC CCAAATTGTT GGAATTATGG 1465

ACATGAGCCA GTGTGCTTGG CCTGATTTTT TTTTTTTTTT TAATGAGAAA AACGTTCCTT 1525

AAGAAAAGTT TCATTGTAAG ACGAGGACTT GCTATGTTGC CAGTTTGGTC TTGAACTCGG 1585

TCTCAAGTGA TTCTCCTGCC TTGGGTTCCC AAAGCGTTTG GGCCGGCAGA TGTCAGCCAC 1645

ACCGCGCCTG CCTTATTCTT ATAAACTCA 1674

(2) INFORMATION FOR SEQ ID NO:8:

(i) SEQUENCE CHARACTERISTICS:
(A) LENGTH: 275 amino acids
(B) TYPE: amino acid

(D) TOPOLOGY: linear

(ii) MOLECULE TYPE: protein

(xi) SEQUENCE DESCRIPTION: SEQ ID NO:8:

Gly Ser Cys Phe Asn Val Glu Ile Lys Arg Asn Asp Asp Leu Ser Trp
1               5                   10                  15

Leu Trp Asn Glu Ser Thr Ala Leu Tyr Pro Ser Ile Tyr Leu Asn Thr
            20                  25                  30

Gln Gln Ser Pro Val Ala Ala Thr Leu Tyr Val Arg Asn Arg Val Arg
        35                  40                  45

Glu Ala Ile Arg Val Ser Lys Ile Pro Asp Ala Lys Ser Pro Leu Pro
    50                  55                  60

Val Phe Ala Tyr Thr Arg Ile Val Phe Thr Asp Gln Val Leu Lys Phe
65                  70                  75                  80

Leu Ser Gln Asp Glu Leu Val Tyr Thr Phe Gly Glu Thr Val Ala Leu
                85                  90                  95

Gly Ala Ser Gly Ile Val Ile Trp Gly Thr Leu Ser Ile Met Arg Ser
            100                 105                 110

Met Lys Ser Cys Leu Leu Leu Asp Asn Tyr Met Glu Thr Ile Leu Asn
        115                 120                 125

Pro Tyr Ile Ile Asn Val Thr Leu Ala Ala Lys Met Cys Ser Gln Val
    130                 135                 140

Leu Cys Gln Glu Gln Gly Val Cys Ile Arg Lys Asn Trp Asn Ser Ser
145                 150                 155                 160

Asp Tyr Leu His Leu Asn Pro Asp Asn Phe Ala Ile Gln Leu Glu Lys
                165                 170                 175

Gly Gly Lys Phe Thr Val Arg Gly Lys Pro Thr Leu Glu Asp Leu Glu
            180                 185                 190

Gln Phe Ser Glu Lys Phe Tyr Cys Ser Cys Tyr Ser Thr Leu Ser Cys
        195                 200                 205

Lys Glu Lys Ala Asp Val Lys Asp Thr Asp Ala Val Asp Val Cys Ile
    210                 215                 220

Ala Asp Gly Val Cys Ile Asp Ala Phe Leu Lys Pro Pro Met Glu Thr
225                 230                 235                 240

Glu Glu Pro Gln Ile Phe Tyr Asn Ala Ser Pro Ser Thr Leu Ser Ala
                245                 250                 255

Thr Met Phe Ile Asp Leu Cys Asp Leu Tyr Leu Val Pro Thr Ser Tyr
            260                 265                 270

Leu Ile Leu
        275

(2) INFORMATION FOR SEQ ID NO:9:

(i) SEQUENCE CHARACTERISTICS:
(A) LENGTH: 1104 base pairs
(B) TYPE: nucleic acid
(C) STRANDEDNESS: double
(D) TOPOLOGY: linear

(ii) MOLECULE TYPE: DNA (genomic)

(xi) SEQUENCE DESCRIPTION: SEQ ID NO:9:

TACTGCACTG GTCAATCTGG AAAGTGCCCT CTAGACACAT ACAAACAAGA TGGTACCCCA 60

TGTAATGAGG GATTCTTCTG TGTAAGTAAG GGGTGCACGG ACCCTGGTAT TCAATGCGCA 120

ACCTATTTTG GGCATGGTGC CAGGTCTGCC CCAGATGCAT GTTACACTAC ATTGAACAGC 180

ATAGGGAATA TATTTGGAAA CTGTGGTCAA TCAGGTAATC CGACCACCTA TGTTGGGTGT 240

TCAGGTGATA GTACAAAGTG TGGGAAACTC ATATGTACAG GTATTTCTTC AATACCTCCA 300

ATCAGAGCTC TATTTGCAGC GATCCAGATC CCTCATGGAG ATGACTGGTG CTGGAGCATT 360

AGTAACTTTG GGGATCCTGC GTCCTCCCCC ACAGAAGGAG CTGTGTCAGC AGGCACGTCT 420

TGCGCTTCAG GCAAAGCGTG TGTAAATGCC CAGTGTTCTA CTTTCACACT TGACACTGCT 480

AACTGTAGTG CAGCTGAAAT GTGTAATGAG AATGGAATTT GCAACAATTT AGGGCACTGC 540

CACTGTGGAG ATGGTTTTGC TCCCCCCAAC TGCAAAGAAC AAGGAACTGG AGGTAGTATA 600

GATAGTGGTC CCCCTCCCCC TTCTAGTACA CCTACTGCAC CTCCTAAACC AACCCAAACG 660

ACAAAAGCAT CAAGTGAAAA CTTAGCATTA ATTGGCCTGA TAATTTTGGT AATACTATTA 720

CTACTTCTAG TTATTTGTGC TATCTGCCTT GGTATACCTG CTGAAGAAGC TCCTCCACCA 780

CCAGAAGAGG AAGAAGCAGG GGAACTGGAA GAAGAACCAG AACCAGAACC AGAACCGGAG 840

GAGGAGGAAG CAGCAGAGGA GGAAGACTAG AGTGATAACT GGTGGAAGGG CAAAGCCAAA 900

TATATCAAAT TCCTCAAGTG ATTACTAGGA ATGAGAAGCT CAGGGAAGCA AAAGTTCAGT 960

GGGAACTGAC AGCTCTAATG GCTCTAACTA GGACTCTATA CTCTAAAAAG ACACCACTGT 1020

AAGAGGACCT ATTCCTCCAT GTTTCTCATC TAAGTAAATA AATTCTTGTT TACCTAGCAT 1080

ATTAAAAACA AAAAAAAAAA AAAA 1104

(2) INFORMATION FOR SEQ ID NO:10:

(i) SEQUENCE CHARACTERISTICS:
(A) LENGTH: 1463 base pairs
(B) TYPE: nucleic acid
(C) STRANDEDNESS: double
(D) TOPOLOGY: linear

(ii) MOLECULE TYPE: DNA (genomic)

(xi) SEQUENCE DESCRIPTION: SEQ ID NO:10:

GAATTCCAGC CCGTCTACAG GTCAAACCCG GTCTGCGGCA ATAACAGGGT GGAACAGGGT 60

GAAGACTGCG ACTGCGGATC GCAGGAGGAA TGCCAAGACA CCTGCTGTGA TGCTGCTACC 120

TGCAGGCTGA AAAGTACTTC ACGATGTGCT CAAGGGCCCT GTTGTAACCA GTGTGAGTTC 180

AAAACTAAAG GAGAGGTATG CCGAGAGTCC ACGGATGAGT GTGATCTCCC TGAGTACTGC 240

AACGGCTCGT CTGGGGCTTG CCAAGAAGAC CTCTATGTTA TTAATGGGCA CAGATGCGCA 300

AATGAAGAGT GGATCTGCAT GAATGGGAGG TGTCTGTCTG GGAAGGCACA ATGCCAAGAA 360

ACATTTGGTA CAGAAATGGA AATGGGTTCA GTAGACTGCT TTGAACAACT TAATACTAAG 420

AATGACATTA CTGGAAACTG TGGTATCCTC AGCCCCGGGA ATTACAAAGC ATGTGGAGCT 480

AGCAATTGGA AGTGTGGAAA ATTAATCTGT TCCTATGATA AGAGCGAAAT CCTGAGAAAC 540

AAGGAAGGCA TGACCATTTA TGCCAACATC AGCGGCCATA TCTGTGTCAG CATAGAATAT 600

CCTCCTGGTC ACGCCAAGAG TGCACTGATG TGGGTAAGAG ATGGCACCGT GTGTGGCCCG 660

AGTGAGGTTT GTAGGCAACA ACAGTGTGTA TCCAGTTCGT ATTTGGGATA TGACTGCACA 720

CCAGCCACTT GCAGTGATCA TGGTGTATGC AATAACAAAA GGCACTGTCA CTGTAATCCC 780

ACCTATGTAC CTCCAAACTG CGAGACCCAA GATTCGACAA AGCCTGGAGG GAGTGTTGAC 840

AGCGGTAATC TACGATATGA ACCAATCCCT GAAACGTATT TCGTTGAAGG TGCTTACCAT 900

ACCAAGTCTA GAAAATGGCC ATTTTTCTTG ATCATTCCTT TTTTCGTTAT TTTCTCCGTA 960

CTGGTTGCTA CAGTGGTAAA AGTCTATTAC CAAAAGAAAA AATGGAAAAC TGAAGATTAT 1020

GCAAATGATG AGAACATTGA AAGCGAGAGT GAACCCAAAA GCTCCAAAGT CTCTTCCAAG 1080

TAGACTGGCT GGCAAGGATT CTATGCTGCC ACAGTGAGTG TCAAATAGTT CAAGTCTTCC 1140

AGAACAAGTA TCTTTAGTGG ATAGAAAAAA GTGGAGAAGA AAAAATATGC ACTACCTTAC 1200

TTCCTGGAAG TCAAACTGAT GTATCGTGGT TCCAGTGCAC CAGAAACTAT AGACATTTCA 1260

TGTACATGTA ACATACATAT ATGTCGTATA TATATGATTC TATAACAGAT AATTTATTTG 1320

TAAGGAAGGC CTAATTATGA GTTTTACATT ACATTTTTCT CATTTTAAAA GTTATTTCAC 1380

ACCGTGTTAG CTAGAAGTCA CTAATTCTGT TAGTAGGCAT GATATAGAAA AAGTTATAAT 1440

AAAGGTAATA CCATGGGAAT TCC 1463

(2) INFORMATION FOR SEQ ID NO:11:

(i) SEQUENCE CHARACTERISTICS:
(A) LENGTH: 40 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:11:

Ser Asn Pro Val Cys Gly Asn Asn Arg Val Glu Gln Gly Glu Asp Cys
1               5                   10                  15

Asp Cys Gly Ser Gln Glu Glu Cys Gln Asp Thr Cys Cys Asp Ala Ala
            20                  25                  30

Thr Cys Arg Leu Lys Ser Thr Ser
        35                  40

(2) INFORMATION FOR SEQ ID NO:12:

(i) SEQUENCE CHARACTERISTICS:
(A) LENGTH: 40 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:12:

Ser Pro Pro Val Cys Gly Asn Glu Leu Leu Glu Ala Gly Glu Glu Cys
1               5                   10                  15

Asp Cys Gly Ser Pro Glu Asn Cys Gln Tyr Gln Cys Cys Asp Ala Ala
            20                  25                  30

Ser Cys Lys Leu His Ser Trp Val
        35                  40

(2) INFORMATION FOR SEQ ID NO:13:

(i) SEQUENCE CHARACTERISTICS:
(A) LENGTH: 40 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:13:

Ser Pro Pro Val Cys Gly Asn Glu Ile Leu Glu Gln Gly Glu Asp Cys
1               5                   10                  15

Asp Cys Gly Ser Pro Ala Asn Cys Gln Asp Gln Cys Cys Asn Ala Ala
            20                  25                  30

Thr Cys Lys Leu Thr Pro Gly Ser
        35                  40

(2) INFORMATION FOR SEQ ID NO:14:

(i) SEQUENCE CHARACTERISTICS:
(A) LENGTH: 27 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:14:

Glu Ala Gly Glu Glu Cys Asp Cys Gly Ser Pro Glu Asn Pro Cys Cys
1               5                   10                  15

Asp Ala Ala Thr Cys Lys Leu Arg Pro Gly Ala
            20                  25

(2) INFORMATION FOR SEQ ID NO:15:

(i) SEQUENCE CHARACTERISTICS:
(A) LENGTH: 25 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:15:

Gly Lys Glu Cys Asp Cys Ser Ser Pro Glu Asn Pro Cys Cys Asp Ala
1               5                   10                  15

Ala Thr Cys Lys Leu Arg Pro Gly Ala
            20                  25

(2) INFORMATION FOR SEQ ID NO:16:

(i) SEQUENCE CHARACTERISTICS:
(A) LENGTH: 50 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:16:

Arg Cys Ala Gln Gly Pro Cys Cys Asn Gln Cys Glu Phe Lys Thr Lys
1               5                   10                  15

Gly Glu Val Cys Arg Glu Ser Thr Asp Glu Cys Asp Leu Pro Glu Tyr
            20                  25                  30

Cys Asn Gly Ser Ser Gly Ala Cys Gln Glu Asp Leu Tyr Val Ile Asn
        35                  40                  45

Gly His
    50

(2) INFORMATION FOR SEQ ID NO:17:

(i) SEQUENCE CHARACTERISTICS:
(A) LENGTH: 50 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:17:

Lys Cys Glu Ser Gly Glu Cys Cys Asp Gln Cys Arg Phe Arg Thr Ala
1               5                   10                  15

Gly Thr Glu Cys Arg Ala Ala Glu Ser Glu Cys Asp Ile Pro Glu Ser
            20                  25                  30

Cys Thr Gly Gln Ser Ala Asp Cys Pro Thr Asp Arg Phe His Arg Asn
        35                  40                  45

Gly Gln
    50

(2) INFORMATION FOR SEQ ID NO:18:

    (i) SEQUENCE CHARACTERISTICS:
        (A) LENGTH: 43 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:18:

Gln Cys Asn His Gly Glu Cys Cys Asp Gln Cys Lys Phe Lys Lys Ala
1               5                   10                  15

Arg Thr Val Cys Arg Ile Ala Arg Gly Asp Trp Asn Asp Asp Tyr Cys
            20                  25                  30

Thr Gly Lys Ser Ser Asp Cys Pro Trp Asn His
        35                  40

(2) INFORMATION FOR SEQ ID NO:19:

    (i) SEQUENCE CHARACTERISTICS:
        (A) LENGTH: 46 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:19:

Gln Cys Ala Asp Gly Leu Cys Cys Asp Gln Cys Arg Phe Met Lys Lys
1               5                   10                  15

Gly Thr Val Cys Arg Val Ala Lys Gly Asp Trp Asn Asp Asp Thr Cys
            20                  25                  30

Thr Gly Gln Ser Ala Asp Cys Pro Arg Asn Gly Leu Tyr Gly
        35                  40                  45

(2) INFORMATION FOR SEQ ID NO:20:

    (i) SEQUENCE CHARACTERISTICS:
        (A) LENGTH: 43 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:20:

Gln Cys Gly Glu Gly Leu Cys Cys Glu Gln Cys Lys Phe Ser Arg Ala
1               5                   10                  15

Gly Lys Ile Cys Arg Ile Pro Arg Gly Asp Met Pro Asp Asp Arg Cys
            20                  25                  30

Thr Gly Gln Ser Ala Asp Cys Pro Arg Tyr His
        35                  40

(2) INFORMATION FOR SEQ ID NO:21:

(i) SEQUENCE CHARACTERISTICS:
(A) LENGTH: 49 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:21:

Glu Cys Glu Ser Gly Pro Cys Cys Arg Asn Cys Lys Phe Leu Lys Glu
1               5                   10                  15

Gly Thr Ile Cys Lys Arg Ala Arg Gly Asp Asp Met Asp Asp Tyr Cys
            20                  25                  30

Asn Gly Lys Thr Cys Asp Cys Pro Arg Asn Pro His Lys Gly Pro Ala
        35                  40                  45

Thr

(2) INFORMATION FOR SEQ ID NO:22:

(i) SEQUENCE CHARACTERISTICS:
(A) LENGTH: 40 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:22:

Ser Asn Pro Val Cys Gly Asn Asn Arg Val Glu Gln Gly Glu Asp Cys
1               5                   10                  15

Asp Cys Gly Ser Gln Glu Glu Cys Gln Asp Thr Cys Cys Asp Ala Ala
            20                  25                  30

Thr Cys Arg Leu Lys Ser Thr Ser
        35                  40

(2) INFORMATION FOR SEQ ID NO:23:

(i) SEQUENCE CHARACTERISTICS:
(A) LENGTH: 40 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:23:

Ser Pro Pro Val Cys Gly Asn Glu Leu Leu Glu Ala Gly Glu Glu Cys
1               5                   10                  15

Asp Cys Gly Ser Pro Glu Asn Cys Gln Tyr Gln Cys Cys Asp Ala Ala
            20                  25                  30

Ser Cys Lys Leu His Ser Trp Val
        35                  40

(2) INFORMATION FOR SEQ ID NO:24:

(i) SEQUENCE CHARACTERISTICS:
(A) LENGTH: 19 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:24:

Tyr Cys Thr Gly Gln Ser Gly Lys Cys Pro Leu Asp Thr Tyr Lys Gln
1               5                   10                  15

Asp Gly Thr

(2) INFORMATION FOR SEQ ID NO:25:

(i) SEQUENCE CHARACTERISTICS:
(A) LENGTH: 50 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:25:

Arg Cys Ala Gln Gly Pro Cys Cys Asn Gln Cys Glu Phe Lys Thr Lys
1               5                   10                  15

Gly Glu Val Cys Arg Glu Ser Thr Asp Glu Cys Asp Leu Pro Glu Tyr
            20                  25                  30

Cys Asn Gly Ser Ser Gly Ala Cys Gln Glu Asp Leu Tyr Val Ile Asn
        35                  40                  45

Gly His
    50

(2) INFORMATION FOR SEQ ID NO:26:

(i) SEQUENCE CHARACTERISTICS:
(A) LENGTH: 50 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:26:

Lys Cys Glu Ser Gly Glu Cys Cys Asp Gln Cys Arg Phe Arg Thr Ala
1               5                   10                  15

Gly Thr Glu Cys Arg Ala Ala Glu Ser Glu Cys Asp Ile Pro Glu Ser
            20                  25                  30

Cys Thr Gly Gln Ser Ala Asp Cys Pro Thr Asp Arg Phe His Arg Asn
        35                  40                  45

Gly Gln
    50

(2) INFORMATION FOR SEQ ID NO:27:

(i) SEQUENCE CHARACTERISTICS:
(A) LENGTH: 49 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:27:

Pro Cys Asn Glu Gly Phe Phe Cys Val Ser Lys Gly Cys Thr Asp Pro
1               5                   10                  15

Gly Ile Gln Cys Ala Thr Tyr Phe Gly His Gly Ala Arg Ser Ala Pro
            20                  25                  30

Asp Ala Cys Tyr Thr Thr Leu Asn Ser Ile Gly Asn Ile Phe Gly Asn
        35                  40                  45

Cys

(2) INFORMATION FOR SEQ ID NO:28:

(i) SEQUENCE CHARACTERISTICS:
(A) LENGTH: 50 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:28:

Arg Cys Ala Asn Glu Glu Trp Ile Cys Met Asn Gly Arg Cys Leu Ser
1               5                   10                  15

Gly Lys Ala Gln Cys Gln Glu Thr Phe Gly Thr Glu Met Glu Met Gly
            20                  25                  30

Ser Val Asp Cys Phe Glu Gln Leu Asn Thr Lys Asn Asp Ile Thr Gly
        35                  40                  45

Asn Cys

(2) INFORMATION FOR SEQ ID NO:29:

(i) SEQUENCE CHARACTERISTICS:
(A) LENGTH: 49 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:29:

Pro Cys Leu Tyr Asn His Gly Tyr Cys Tyr Asn Gly Lys Cys Pro Ile
1               5                   10                  15

Met Phe Tyr Gln Cys Tyr Phe Leu Phe Gly Ser Asn Ala Thr Val Ala
            20                  25                  30

Glu Asp Asp Cys Phe Asn Asn Asn Lys Lys Gly Asp Lys Tyr Phe Tyr
        35                  40                  45

Cys

(2) INFORMATION FOR SEQ ID NO:30:

(i) SEQUENCE CHARACTERISTICS:
(A) LENGTH: 50 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:30:

Gly Gln Ser Gly Asn Pro Thr Thr Tyr Val Gly Cys Ser Gly Asp Ser
1               5                   10                  15

Thr Lys Cys Gly Lys Leu Ile Cys Thr Gly Ile Ser Ser Ile Pro Pro
            20                  25                  30

Ile Arg Ala Leu Phe Ala Ala Ile Gln Ile Pro His Gly Asp Asp Trp
        35                  40                  45

Cys Trp
    50

(2) INFORMATION FOR SEQ ID NO:31:

(i) SEQUENCE CHARACTERISTICS:
(A) LENGTH: 49 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:31:

Gly Ile Leu Ser Pro Gly Asn Tyr Lys Ala Cys Gly Ala Ser Asn Trp
1               5                   10                  15

Lys Cys Gly Lys Leu Ile Cys Ser Tyr Asp Lys Ser Glu Ile Leu Arg
            20                  25                  30

Asn Lys Glu Gly Met Thr Ile Tyr Ala Asn Ile Ser Gly His Ile Cys
        35                  40                  45

Val

(2) INFORMATION FOR SEQ ID NO:32:

(i) SEQUENCE CHARACTERISTICS:
(A) LENGTH: 30 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:32:

Arg Lys Glu Asn Glu Lys Tyr Ile Pro Cys Ala Gln Glu Asp Val Lys
1               5                   10                  15

Cys Gly Arg Leu Phe Cys Asp Asn Lys Lys Tyr Pro Cys His
            20                  25                  30

(2) INFORMATION FOR SEQ ID NO:33:

(i) SEQUENCE CHARACTERISTICS:
(A) LENGTH: 50 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:33:

Ser Ile Ser Asn Phe Gly Asp Pro Ala Ser Ser Pro Thr Glu Gly Ala
1               5                   10                  15

Val Ser Ala Gly Thr Ser Cys Ala Ser Gly Lys Ala Cys Val Asn Ala
            20                  25                  30

Gln Cys Ser Thr Phe Thr Leu Asp Thr Ala Asn Cys Ser Ala Ala Glu
        35                  40                  45

Met Cys
    50

(2) INFORMATION FOR SEQ ID NO:34:

(i) SEQUENCE CHARACTERISTICS:
(A) LENGTH: 48 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:34:

Ser Ile Glu Tyr Pro Pro Gly His Ala Lys Ser Ala Leu Met Trp Val
1               5                   10                  15

Arg Asp Gly Thr Val Cys Gly Pro Ser Glu Val Cys Arg Gln Gln Gln
            20                  25                  30

Cys Val Ser Ser Ser Tyr Leu Gly Tyr Asp Cys Thr Pro Ala Thr Cys
        35                  40                  45

(2) INFORMATION FOR SEQ ID NO:35:

(i) SEQUENCE CHARACTERISTICS:
(A) LENGTH: 38 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:35:

Tyr Asn Tyr Ser Glu Asp Leu Asp Phe Gly Met Val Asp His Gly Thr
1               5                   10                  15

Lys Cys Ala Asp Gly Lys Val Cys Ser Asn Arg Gln Cys Val Asp Val
            20                  25                  30

Asn Glu Ala Tyr Lys Ser
        35

(2) INFORMATION FOR SEQ ID NO:36:

(i) SEQUENCE CHARACTERISTICS:
(A) LENGTH: 47 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:36:

Asn Glu Asn Gly Ile Cys Asn Asn Leu Gly His Cys His Cys Gly Asp
1               5                   10                  15

Gly Phe Ala Pro Pro Asn Cys Lys Glu Gln Gly Thr Gly Gly Ser Ile
            20                  25                  30

Asp Ser Gly Pro Pro Pro Pro Ser Ser Thr Pro Thr Ala Pro Pro
        35                  40                  45

(2) INFORMATION FOR SEQ ID NO:37:

(i) SEQUENCE CHARACTERISTICS:
(A) LENGTH: 50 amino acids
(B) TYPE: amino acid
(C) STRANDEDNESS: single

(D) TOPOLOGY: linear

(ii) MOLECULE TYPE: peptide

(xi) SEQUENCE DESCRIPTION: SEQ ID NO:37:

Ser Asp His Gly Val Cys Asn Asn Lys Arg His Cys His Cys Asn Pro
1               5                   10                  15

Thr Tyr Val Pro Pro Asn Cys Glu Thr Gln Asp Ser Thr Lys Pro Gly
            20                  25                  30

Gly Ser Val Asp Ser Gly Asn Leu Arg Tyr Glu Pro Ile Pro Glu Thr
        35                  40                  45

Tyr Phe
    50

(2) INFORMATION FOR SEQ ID NO:38:

(i) SEQUENCE CHARACTERISTICS:
(A) LENGTH: 50 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:38:

Lys Pro Thr Gln Thr Thr Lys Ala Ser Ser Glu Asn Leu Ala Leu Ile
1               5                   10                  15

Gly Leu Ile Ile Leu Val Ile Leu Leu Leu Leu Leu Val Ile Cys Ala
            20                  25                  30

Ile Cys Leu Gly Ile Pro Ala Glu Glu Ala Pro Pro Pro Pro Glu Glu
        35                  40                  45

Glu Glu
    50

(2) INFORMATION FOR SEQ ID NO:39:

(i) SEQUENCE CHARACTERISTICS:
(A) LENGTH: 50 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:39:

Val Glu Gly Ala Tyr His Thr Lys Ser Arg Lys Trp Pro Phe Phe Leu
1               5                   10                  15

Ile Ile Pro Phe Phe Val Ile Phe Ser Val Leu Val Ala Thr Val Val
            20                  25                  30

Lys Val Tyr Tyr Gln Lys Lys Lys Trp Lys Thr Glu Asp Tyr Ala Asn
        35                  40                  45

Asp Glu
    50

(2) INFORMATION FOR SEQ ID NO:40:

     (i) SEQUENCE CHARACTERISTICS:
          (A) LENGTH: 24 amino acids
          (B) TYPE: amino acid
          (C) STRANDEDNESS: single
          (D) TOPOLOGY: linear

    (ii) MOLECULE TYPE: peptide

    (xi) SEQUENCE DESCRIPTION: SEQ ID NO:40:

     Ala Gly Glu Leu Glu Glu Glu Pro Glu Pro Glu Pro Glu Pro Glu Glu
     1               5                   10                  15

     Glu Glu Ala Ala Glu Glu Glu Asp
                 20

(2) INFORMATION FOR SEQ ID NO:41:

     (i) SEQUENCE CHARACTERISTICS:
          (A) LENGTH: 16 amino acids
          (B) TYPE: amino acid
          (C) STRANDEDNESS: single
          (D) TOPOLOGY: linear

    (ii) MOLECULE TYPE: peptide

    (xi) SEQUENCE DESCRIPTION: SEQ ID NO:41:

     Asn Ile Glu Ser Glu Ser Glu Pro Lys Ser Ser Lys Val Ser Ser Lys
     1               5                   10                  15

(2) INFORMATION FOR SEQ ID NO:42:

     (i) SEQUENCE CHARACTERISTICS:
          (A) LENGTH: 23 amino acids
          (B) TYPE: amino acid
          (C) STRANDEDNESS: single
          (D) TOPOLOGY: linear

    (ii) MOLECULE TYPE: peptide

    (xi) SEQUENCE DESCRIPTION: SEQ ID NO:42:

      Gly Ala Asp Thr Arg Cys Gly Arg Leu Ile Cys Gly Leu Ser Thr Thr      1
                  5                   10                  15

      Ala Pro Thr Arg Gln Tyr Pro
                  20

(2) INFORMATION FOR SEQ ID NO:43:

     (i) SEQUENCE CHARACTERISTICS:
          (A) LENGTH: 8 amino acids
          (B) TYPE: amino acid
          (C) STRANDEDNESS: single
          (D) TOPOLOGY: linear

(ii) MOLECULE TYPE: peptide

(xi) SEQUENCE DESCRIPTION: SEQ ID NO:43:

Ser Thr Asp Glu Cys Asp Leu Lys
1               5

(2) INFORMATION FOR SEQ ID NO:44:

(i) SEQUENCE CHARACTERISTICS:
(A) LENGTH: 6 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:44:

Cys Ser Thr Asp Glu Cys
1               5

(2) INFORMATION FOR SEQ ID NO:45:

(i) SEQUENCE CHARACTERISTICS:
(A) LENGTH: 6 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:45:

Gly Arg Gly Asp Thr Pro
1               5

(2) INFORMATION FOR SEQ ID NO:46:

(i) SEQUENCE CHARACTERISTICS:
(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:46:

Gly Arg Gly Glu Ser
1               5

## Description

### Background

Immunization of male and female animals with extracts of whole sperm cells is known to cause infertility (Tung, K., et al., J. of Reproductive Immunol., 1: 145-158 (1979) and Menge, A., et al., Biol. of Reproduction, 20: 931-937 (1979)). Also, men and women who spontaneously produce antisperm antibodies are infertile, but otherwise healthy (Bronson, R. et al., Fert. and Steril., 42: 171-183 (1984)). Although the critical sperm antigens are unknown, these observations have led to the proposal that sperm proteins might be useful in the development of a contraceptive vaccine.

In mammalian species, sperm proteins have been proposed to have a role in sperm adhesion to the zona pellucida of the egg. In the mouse, it has been shown that a sperm surface galactosyl transferase is an adhesion protein that functions in acrosome-intact sperm binding to the zona (Shur, B.E., Galactosyl transferase as a recognition molecule during fertilization and development, In: "The Molecular Biology of Fertilization," Eds. Schatten, H., and Schatten, G., Academic Press, pps. 37-71 (1989)). On rat sperm, there is a galactose receptor, (RTG-r), related to the hepatic asialoglycoprotein receptor, which could function through its lectin properties in sperm binding to zona oligosaccharides (Abdullah, M., and Kierszenbaum, A.L., J. Cell Biol., 108: 367-375 (1989)). A boar sperm plasma membrane protein (AP_{z}), distinct from galactosyl transferase, and a rabbit sperm protein have also been reported to have a role in sperm-zona adhesion (Peterson, R.N. and Hunt, W.P., Gam. Res., 23: 103-118 (1989) and O'Rand, M.G., et at., Dev. Biol., 129: 231-240 (1988)).

The guinea pig sperm surface protein PH-20 has been shown to have a required function in sperm adhesion to the extracellular coat (zona pellucida) of the egg, a necessary initial step in fertilization. In male and female guinea pigs immunized with PH-20, 100% effective contraception was obtained. Antisera from immunized females had high titers, specifically recognized PH-20 in sperm extracts and blocked sperm adhesion to egg zona pellucida, in vitro. The contraceptive effect was long-lasting and reversible; immunized females mated at intervals of 6-15 months after immunization progressively regained fertility. A second guinea pig sperm surface protein, PH-30, has also shown a contraceptive effect.

WO 92/10569 (international publication date 25 June 1992) discloses the DNA sequences of genes encoding guinea pig, mouse and human PH-20 proteins. It was found that portions of the isolated guinea pig PH-20 gene used as probes would hybridise to DNA fragments of each of several mammalian genomic DNA digests tested in a Southern hybridization.

In WO 93/06859 (international publication date 15 April 1993) the t-complex associated testes expressed-1 (tcte-1) cDNAs of mouse and human were each isolated and expressed in *E. coli* as fusion proteins. Human and mouse genes encoding this sperm surface protein were noted to be highly conserved. When the mouse cDNA was used as a probe in a Southern hybridization, it was found to hybridize to genomic DNA of *Xenopus*, chicken and several mammals. Preincubation of rabbit anti-(mouse)-Tcte-1 IgG with mouse sperm reduced the proportion of sperm capable of binding to mouse eggs.

Other sperm proteins tested as contraceptive immunogens include the sperm enzymes hyaluronidase, acrosin and lactate dehydrogenase C-4. Immunization of female animals with these enzymes had either no effect on fertility or partial effects on fertility, which were not large enough to make these proteins suitable as contraceptive agents. The high contraceptive effectiveness of PH-20 in the guinea pig, seems to depend on several of its specific properties, including its presence on the sperm surface, its strong immunogenicity and its essential role in fertilization.

Mammalian sperm-zona adhesion is in most cases species specific. Sperm from other mammalian species are like guinea pig sperm in that they can bind to the zona pellucida either before or after the acrosome reaction. The identification and isolation of sperm surface proteins essential for fertilization in species other than guinea pig would be useful for developing vaccines for effective immunization and providing long lasting contraception in those species. The lack of biochemical identification, isolation and cloning of candidate adhesion proteins of sperm has hindered scientists in developing effective contraceptive vaccines for humans as well as other mammalian species.

### Summary of the Invention

The subject invention relates to methods of contraception in which one of more PH-30 sperm surface proteins are administered to a mammal. The sperm surface protein (or portion of same) is administered in an amount effective for the stimulation of an immune response whereby antibody which binds to the sperm surface protein is produced in a titer sufficient to prevent or substantially reduce the rate of sperm-egg fusion. The protein, or protein fragment, can be purified by conventional methods or produced by recombinant DNA methodology.

The invention also relates to contraceptive compositions for use in the methods described herein. These compositions contain a sperm surface protein, or a portion thereof. Preferred sperm surface proteins are PH-30 sperm surface proteins.

### Brief Description of the Drawings

Figure 1 is a diagram representing a partial restriction map of DNA encoding the guinea pig PH-20 protein, and the relative positions of 5 cDNA clones.
Figure 2 is a diagram representing the guinea pig cDNA sequence encoding the PH-20 protein, and the reduced amino acid sequence of the guinea pig PH-20 protein presented in single letter code.
Figure 3 is a diagram representing the murine DNA sequence encoding the PH-20 protein. The sequence disclosed in Figure 3 is represented as SEQ ID NO 3 and 4.
Figure 4 is a diagram representing the human DNA sequence encoding one form of the human PH-20 protein, and the deduced amino acid sequence presented in three letter code. The sequence disclosure of Figure 4 is represented as SEQ ID NO 5 and 6.
Figs. 5A-B are diagrams representing the human DNA sequence encoding a portion of a second form of the human PH-20 protein, and the deduced amino acid sequence presented in three letter code. The sequence disclosure of Figure 5 is represented as SEQ ID No 7 and 8.
Figure 6 is a diagram representing the present invention with the guinea pig PH-30 α subunit DNA sequence. The sequence disclosure of Figure 6 is represented as SEQ ID NO 9.
Figure 7 is a diagram representing the present invention with the guinea pig PH-30 β subunit DNA sequence. The sequence disclosure of Figure 7 is represented as SEQ ID NO 10.
Figure 8A-B are diagrams representing the present invention with a comparison of amino acid sequences of guinea pig PH-30 with disintegrin sequences. Figure 8 lists 31 peptide sequences in total. These sequences are represented consecutively in the Sequence Listing as SEQ ID NO 11 through SEQ ID NO 41.

### Detailed Description of the Invention

The subject invention relates to PH-30 sperm surface proteins which are essential for fertilization, or portions thereof, and their use in contraceptive methods. A sperm surface protein is essential for fertilization if, for example, a monoclonal antibody to the protein or a polyclonal antibody raised against the purified protein, when bound to sperm, inhibits in vitro or in vivo fertilization or any step of in vitro fertilization. The process of fertilization is defined as the binding or fusion of two gametes (sperm and egg) followed by the fusion of their nuclei to form the genome of a new organism. The surface protein can be located in the plasma membrane of sperm and/or the inner acrosomal membrane, it can be a protein or glycoprotein. The isolated surface protein used for immunization can comprise the entire surface protein or some portion of the protein (external to the cell) which is immunogenic.

The present invention relates to PH-30 proteins and all references to PH-20 are for information only.

Both the PH-20 and PH-30 genes encode proteins which are present on the surface of sperm cells and are essential for fertilization. It has been determined that the DNA encoding PH-20 in one mammalian species is cross-reactive (i.e., hybridizable) with genomic DNA from all other mammals tested. The existence of these homologues in other mammalian species was an unexpected finding since mammalian sperm-zona pellucida adhesion is, in most cases, species specific. Similarly, guinea pig PH-30 cDNA hybridizes with human genomic DNA.

Female guinea pigs immunized with affinity-purified guinea pig PH-20 were completely infertile for at least six months, and their sera blocked sperm binding to the egg zona pellucida *in vitro*. A small number of male guinea pigs immunized with guinea pig PH-20 were infertile for at least seven months (Primakoff, P., et al., *Nature 335*:543-546 (1988)).

The deduced amino acid sequences of the α and β subunits of guinea pig PH-20 reveal similarities to each other, suggesting evolutionary relatedness, and suggesting that both are type-I integral membrane proteins with large N-terminal extracellular domains and short cytoplasmic tails. The β subunit contains a putative integrin-binding "disintegrin" domain that could function in binding a putative receptor (integrin, or integrin-like) on the egg plasma membrane (Blobel, C.P., et al., *Nature 356*:248-252 (1992)).

### Production and Purification of Immunogen

A preferred method for producing sperm surface proteins for use as a contraceptive immunogen is by recombinant DNA technology. To produce the protein using this technology it is necessary to isolate and clone DNA encoding the protein, or an immunogenic portion thereof. Those skilled in the art are familiar with a variety of approaches which can be used in an effort to clone a gene of interest. However, having nothing more than the isolated protein of interest, success in such an effort can not be predicted with a reasonable degree of certainty.

In Reference Example 1 which follows, Applicants' report the isolation and cloning of DNA encoding the guinea pig PH-20 gene. The method used to isolate DNA encoding the 3' portion of the PH-20 gene involved the screening of a cDNA expression library with polyclonal sera reactive with the PH-20 protein. Anchored PCR was used to isolate the 5' portion of the gene.

Reference Example 2 reports the surprising finding that a broad spectrum of mammalian genomic DNA contains DNA sequences which hybridize to guinea pig PH-20 sequences under the hybridization conditions described. In fact, cross-reacting sequences were identified in each of the mammalian samples analyzed.

The information presented in Reference Examples 1 and 2, enable one skilled in the art to isolate and clone the PH-20 gene from any mammalian species. For example, a cDNA library is prepared from testis or spermatogenic cells isolated from a mammal of interest (e.g., feline, equine, canine, bovine, etc.). This can be a time consuming process, but it is technically straightforward. One skilled in the art would approach this task with a high degree of certainty with regard to success.

Such a cDNA library is then screened using, for example, labeled guinea pig PH-20 DNA probes. DNA encoding all or a portion of PH-20 is characterized by the ability to hybridize to such a probe sequence under hybridization conditions such as those described in Reference Example 2. Methods of labeling and screening by hybridization are very well known in the art. Positive clones are analyzed, and a full length cDNA is constructed by conventional methods. In light of Applicants' teaching that each of the 7 mammals analyzed contained cross-hybridizing sequences, one skilled in the art would expect all mammals to contain cross-hybridizing species. It is this methodology which enabled Applicants to isolate and clone the murine and human PH-20 genes, as described in greater detail below.

The cloned gene, or portions thereof which encode an immunogenic region of the PH-20 protein, can be expressed by inserting the coding region into an expression vector to produce an expression construct. Many such expression vectors are known to those skilled in the art. These vectors contain a promoter for the gene of interest as well as additional transcriptional and translational signals. Expression vectors for both eukaryotic host cells and prokaryotic host cells are widely available. The DNA expression construct is used to transform an appropriate host cell.

Eukaryotic, in particular mammalian, host cells are preferred for the expression of the sperm surface protein. It has been found, for example, that eukaryotic proteins frequently exhibit folding problems when expressed in prokaryotic cells. In addition, production of authentic, biologically active eukaryotic proteins from cloned DNA frequently requires post-translational modification such as disulfide bond formation, glycosylation, phosphorylation or specific proteolytic cleavage processes that are not performed in bacterial cells. This is especially true with membrane proteins. The sperm surface protein is produced using the transcriptional and translational components of the host cell. After an appropriate growth end expression period, the host cell culture is lysed and the sperm surface protein is purified from the lysate. Lysis buffers typically include non-ionic detergent, protease inhibitors, etc.

From the solubilized cell extract, the sperm surface protein can be purified and isolated by physical and biochemical methods such as ultracentrifugation, column chromatography, high performance liquid chromatography, electrophoresis, etc. Alternatively, the sperm surface protein can be isolated by affinity chromatography using monoclonal or polyclonal antibodies (see Primakoff et al., Biol. of Reprod. 38: 921-934 (1988)). Such methods for purifying proteins are well known to those skilled in the art.

The methods outlined above with respect to the PH-20 gene and gene product are also applicable to the PH-30 gene and gene products according to the present invention. The isolation of DNA encoding the α and β subunits of the PH-30 sperm surface protein is described in Example 5. Methods analogous to those described above can be used to isolate and express mammalian homologues to the guinea pig PH-30 protein. It can be predicted with a high degree of certainty that DNA encoding the guinea pig PH-30 sperm surface protein can be used as a probe to isolate homologous sequences from other mammals. This prediction is based on the fact that guinea pig DNA has been used to identify the presence of a PH-30 homologue in human genomic DNA.

As mentioned above, antigenic portions of the sperm surface protein are useful as immunogen, in addition to the full length protein. Antigenic fragments can be produced, for example, by proteolytic digestion of the full length protein, followed by isolation of the desired fragment. Alternatively, chemical synthesis can be used to generate the desired fragment starting with monomer amino acid residues.

With respect to the PH-30 protein according to the present invention, certain antigenic domains are preferred candidates for use in a contraceptive vaccine. As is discussed in greater detail in the Exemplification section which follows, the PH-30 β subunit contains a domain which is highly conserved when compared to a class of proteins known as disintegrins. A peptide (or portion thereof) which is identical or substantially identical to this domain is preferred for use in the contraceptive methods of this invention. Substantially identical, as used in the preceding sentence, means that at least 80% of the amino acid sequence of the peptide is identical to the corresponding portion of the PH-30 β disintegrin domain.

Disintegrins are found in snake venom, for example, and are known to bind to a class of platelet surface proteins known as integrins. The binding of disintegrins to integrins has been shown to inhibit blood clotting. By analogy, peptides corresponding to the PH-30 β disintegrin domain are predicted to be active in sperm-egg binding and fusion.

### Contraceptive Vaccine

Once the sperm surface protein has been produced and purified, a vaccine can be produced by combining the sperm surface protein with a suitable carrier for administration to a subject for immunization. A vaccine can contain one or more sperm surface proteins. Sperm surface proteins used in the present invention can be combined with adjuvants which contain non-specific stimulators of the immune system. Proper use of adjuvants can induce a strong antibody response to foreign antigens (i.e., sperm surface proteins). The action of adjuvants is not fully understood, but most adjuvants incorporate two components. One is a substance designed to form a deposit which protects the antigen from catabolism. Two methods of forming a deposit are to use mineral oils or aluminum hydroxide precipitates. With mineral oils, such as Freund's adjuvant, the immunogen is prepared in a water-in-oil emulsion. For aluminum hydroxide, the immunogen is either adsorbed to preformed precipitants or is trapped during precipitation. Alternative delivery systems include liposomes or synthetic surfactants. Liposomes are only effective when the immunogen is incorporated into the outer lipid layer; entrapped molecules are not seen by the immune system.

The second component required for an effective adjuvant is a substance that will stimulate the immune system nonspecifically. These substances stimulate the production of a large set of soluble peptide factors known as lymphokines. In turn, lymphokines stimulate the activity of antigen-processing cells directly and cause a local inflammatory reaction at the site of injection. A component of lipopolysaccharide known as lipid A is commonly used. Lipid A is available in a number of synthetic and natural forms that are much less toxic than lipopolysaccharides, but still retain most of the desirable adjuvant properties of the lipopolysaccharide molecules. Lipid A compounds are often delivered using liposomes. The two bacteria that are commonly used in adjuvants as non-specific stimulants are Bordatella pertussis and Mycobacterium tuberculosis. When used as whole bacteria, they must be heat-killed prior to use. The immunomodulatory mediators of B. pertussis include a lipopolysaccharide component and the pertussis toxin. The pertussis toxin has been purified and is available commercially. M. tuberculosis is commonly found in complete Freund's adjuvant. The most active component of M. tuberculosis has been localized to muramyl dipeptide which is available in a number of forms.

### Immunizations (Inoculation and Booster Shots)

The subject to be immunized can be any mammal which possesses a competent immune system. Examples of subject mammals include humans and domestic animals (e.g., dogs, cats, cows, horses, etc.), as well as animals intended for experimental or other purposes (e.g., mice, rats, rabbits, etc.).

Two different criteria are important to consider in determining the proper dose for the initial immunization. First, the optimum dose to achieve the strongest response and second, the minimum dose likely to induce the production of useful polyclonal antibodies. Much of the injected material will be catabolized and cleared before reaching the appropriate target immune cell. The efficiency of this process will vary with host factors, the route of injection, the use of adjuvants, and the intrinsic nature of the surface protein injected. Thus, the effective dose delivered to the immune system may bear little relationship to the introduced dose and consequently dose requirements must be determined empirically. These determinations can be readily made by one skilled in the art. Secondary injections and later boost can be given with amounts similar to or less than the primary injection.

The route of injection is guided by three practical decisions: 1) what volume must be delivered; 2) what buffers and other components will be injected with the immunogen; and 3) how quickly the immunogen should be released into the lymphatics or circulation. For example, with rabbits, large volume injections normally are given at multiple subcutaneous sites. For mice, large volumes are only possible with intraperitoneal injections. If adjuvants or particulate matter are included in the injection, the immunogen should not be delivered intravenously. If a slow release of the inoculant is desired, the injections should be done either intramuscularly or intradermally. For immediate release, use intravenous injections.

Primary antibody responses often are very weak, particularly for readily catabolized, soluble antigens. Hence, secondary or booster injections are required after the initial immunization. A delay is needed before reintroducing the protein into a primed subject. A minimum of 2 or 3 weeks is recommended but greater intervals are possible. The antibody responses to secondary and subsequent injections is much stronger. Higher titers of antibody are reached, but more importantly, the nature and quantity of the antibodies present in serum changes. These changes yield high-affinity antibodies. The intervals between secondary, tertiary and subsequent injections may also be varied, but usually need to be extended to allow the circulating level of antibody to drop enough to prevent rapid clearance of newly injected antigen.

Subsequent booster injections will be required to increase reduced circulating antibody for continued contraception. The actual intervals for these injections will differ from species to species. However, the intervals can be determined by one skilled in the art by monitoring serum levels of sperm surface protein antibodies.

In another embodiment, subjects can be administered alloantisera, or monoclonal antibodies, directed to a sperm surface protein to achieve contraception. The alloantiserum is raised in another individual of the same species, isolated from the serum of the individual and prepared in a suitable carrier for injection into the recipient subject. Those skilled in the art are familiar with methods for preparing and formulating monoclonal antibodies for administration.

The present invention is further explained in the following exemplification.

### EXAMPLES

### Reference Example 1: Isolation of DNA Encoding Guinea Pig PH-20 Library construction and screening

A population of guinea pig testicular cells, enriched for spermatogenic cells on a Percoll gradient was used for the isolation of spermatogenic cell total RNA. The pelleted cells were lysed with detergent in the presence of vanadyl-ribonucleoside complexes (VRC) in 0.5-1.0 ml of solution containing 10 mM Tris (pH 8.6), 0.5% NP-40, 0.14 M NaCl, 1.5 mM MgCl₂ and 10 mM VRC. After pelleting cellular debris, 0.5 volume of 2X Proteinase K buffer (2X=0.2 M Tris (pH 7.5), 25 mM EDTA (pH 8.0), 0.3 M NaCl, and 2.0% SDS) and 200 µg/ml Proteinase K was added to the supernant. PolyA+ RNA was purified from the total RNA by oligo-dT cellulose chromatography. cDNA was synthesized using standard methods. Size selected cDNA (0.5-7kb) was ligated with lambda gt11 arms and packaged into lambda coat proteins, utilizing kits and protocols from Amersham Corporation.

The unamplified library was plated at 20,000 plaques/150 mm plate for screening. A single nitrocellulose filter from each plate was immunoblotted with rabbit anti-PH-20 polyclonal antiserum, raised against affinity-purified PH-20 protein (Primakoff et al., Biol. Reprod. 38:921-934 (1988)), and diluted 1/500 in TBST (10mM Tris (pH 8.0), 0.15 M NaCl, 0.05% Tween-20) containing 2 mg/ml E. coli protein. The E. coli protein was prepared by pelleting an overnight culture of Y1090 cells, resuspending the cells in a minimal volume of TBST and freezing in liquid nitrogen. The thawed cells were sonicated and the protein concentration determined using the BCA reagent (Pierce chemical). Six positive plaques were detected with an anti-rabbit IgG alkaline phosphatase-conjugated second antibody (Promega Biotec). Size of the fusion protein made by plaque-purified positive clones was determined to vary between 118-157 kD as determined by the analysis of E. coli extracts containing the fusion protein on SDS-PAGE. Inserts from the six positive clones were subcloned into pUC19 and sequenced at least partially.

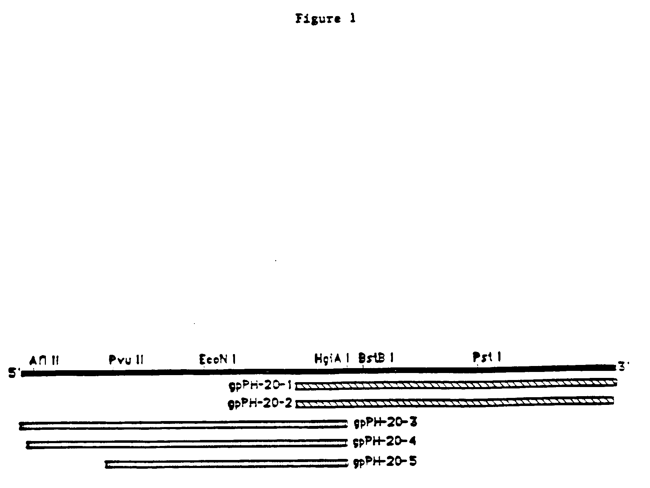

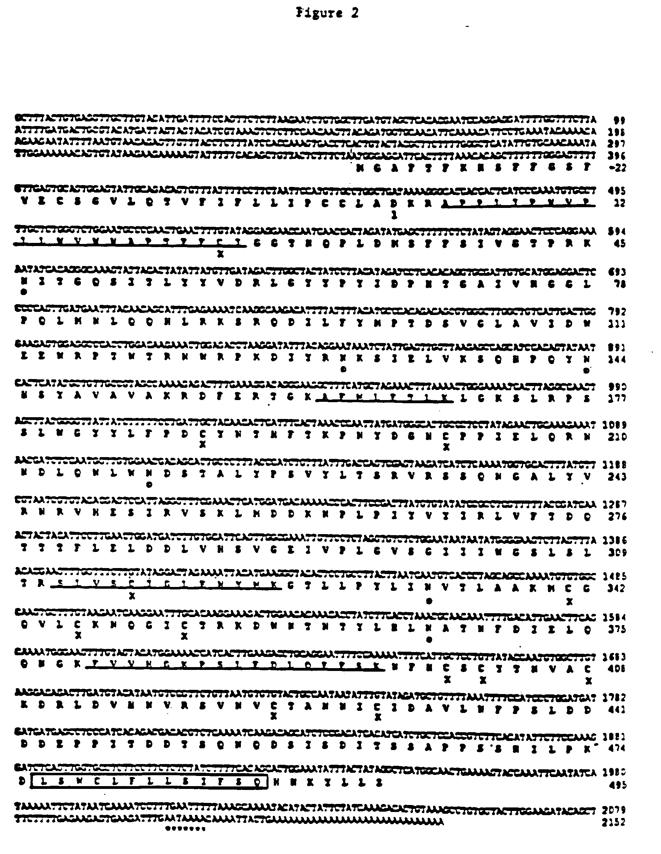

Two of the inserts were confirmed to code for the PH-20 protein by locating the sequences of two PH-20 tryptic peptides in their derived amino acid sequence. Both of these inserts (gpPH-20-1, nucleotide (nt) 1016-2152 and gpPH-20-2, nt 1010-2125, Figure 1 and 2) contained a long (-925 nt) open reading frame, a stop codon, a 3' untranslated region and a polyA tail. Thus these two inserts were concluded to represent the 3' end of a cDNA for PH-20. The other four antibody-positive lambda clones were unrelated to PH-20.

The 5' portion of the PH-20 cDNA was cloned utilizing anchored PCR following the protocol of Frohman et al., (Proc. Natl. Acad. Sci. USA 85: 8998-9002 (1988)). PolyA+ RNA from spermatogenic cells (2µg in 10 µl dH₂O) was heated to 65°C for 3 min and then reverse transcribed by adding 4 µl 10 X RTC buffer (1X buffer is 50 mM Tris (pH 8.3), 50 mM KCl, 4 mM dithiothreitol, 10 mM MgCl₂)), and 4 µl 10 mM stock of each dNTP (1 mM final), 2 µl of 80 mM sodium pyrophosphate (4 mM final) , 1 µl (40 units) of RNasin (Promega Biotec), 40 pmol PH-20 specific primer (PH-20-RT), 18 units AMV reverse transcriptase (Life Sciences) and 40 µCi ³²P-dCTP in 40 µl total volume. After 1 hour of incubation at 42°C, an additional 1 µl of reverse transcriptase was added and incubation continued for a second hour. The PH-20-RT primer was a 17 nucleotide (nt) oligomer (nt 1242-1258, Figure 2), -250 bases downstream from the 5' end of the insert gpPH-20-1 (Figure 1).

The single strand cDNA was separated from excess PH-20-RT by column chromatography, tailed with polyA and diluted to 1.0 ml. Second strand synthesis and PCR amplification were performed with a GeneAmp kit (Perkin Elmer Cetus) in a 100 µl reaction containing 10 µl of the reverse transcription product, 20 pmol (dT) 17 adapter, 50 pmol adapter and 50 pmol PH-20-AMP primer. The PH-20-AMP primer was a 17 nt oligomer (nt 1202-1218, Figure 2) located upstream from the PH-20-RT primer. The PCR product was purified from unincorporated primers and free nucleotide by spin column chromatography (columns from Boehringer-Mannheim). It was subsequently digested with HgiA I and Sal I, gel purified and ligated into pBluescript digested with Pst I and Sal I. The major PCR product was 1.2 kb, and Southern Blot analysis confirmed that this band hybridized with the labeled insert gpPH-20-1. The major PCR products from three separate reactions were cloned and one insert from each of the three reactions was sequenced (gpPH-20-3, nt 1-1175, gpPH-20-4, nt 24-1175 and gpPH-20-5, nt 295-1175).

The complete cDNA sequence and the deduced amino acid sequence were obtained from the five cDNA inserts (Figure 2) that were sequenced in their entirety on both strands. The cDNA sequence contains a 354 nt 5' untranslated region, a 1590 nt open reading frame, and a 208 nt 3' untranslated region. The derived amino acid sequence contains all the tryptic peptide sequences obtained from purified PH-20, confirming that the cDNAs are authentic PH-20 clones. Hybridization experiments indicated that guinea pig genomic DNA contained a single gene for PH-20. Computer searches revealed no significant homology of the guinea pig PH-20 amino acid sequence with other known sequences.

### Reference Example 2: PH-20 Homologues in Other Mammalian Species

To determine if there is a homologue of the PH-20 gene in the genomic DNA of other species, cross species Southern blots were performed. Genomic DNA was isolated from guinea pig, rat, rabbit, mouse, and hamster spleens by detergent lysis-Proteinase K digestion. Other DNA samples (i.e., human, monkey and chicken) were provided by other investigators at the University of Connecticut Health Center. DNA from salmon sperm and bovine thymus were purchased from Sigma and reconstituted at 1 mg/ml in TE (10 mM Tris (pH 8.0), 1 mM EDTA (pH 8.0)). All species DNA's (10µg) were cut with restriction enzymes and separated on a 1% agarose gel. The Southern transfer was carried out by capillary transfer onto nylon membrane. The membranes were prehybridized in a solution consisting of 6XSSC, 1X Denhardt's, 250 mg/ml salmon sperm DNA, 1% SDS, and 50 mM NaPO₄ (pH 7.4), for 1-2 hours at 65°C. The membranes were hybridized overnight at 55°C in prehybridization buffer plus 2 x 10⁶ cpm/ml probe. Probes were prepared by the random hexamer method. The blot was washed 3 X 5 min in 2XSSC + 1.0% SDS at room temperature, 2 X 30 min in 2XSSC + 0.1% SDS at room at 50°C, and 2 X 30 min in 1XSSC + 0.1% SDS at 60°C. The blot was wrapped in plastic wrap and exposed to film with an intensifying screen at -70°C.

The blots were probed with a mix of labeled gpPH-20-3 and gpPH-20-2. The Southern blots exhibited a weakly hybridizing band at -10 kb for chicken DNA and strongly hybridizing bands for mouse, rat, hamster, rabbit and human DNA. In addition, hybridization was observed with bovine and monkey DNA.

### Reference Example 3: Isolation of DNA Encoding Mouse PH-20

PolyA+ RNA was isolated from murine round spermatids and used to produce a cDNA library in lambda J using conventional methods. The library was screened using a labeled full length guinea pig PH-20 cDNA probe. The probe was produced by first isolating guinea pig PolyA+ RNA. An oligo-dT primer was hybridized to the poly(A) tract and reverse transcriptase was used to generate a first cDNA strand. Two oligonucleotides, a first being complementary to a portion of the guinea pig PH-20 5' untranslated region and a second being complementary to the 3' untranslated region, were added to the reaction mixture and a full length double stranded DNA sequence containing the entire coding region was generated by polymerase chain reaction. The product of this reaction was a double stranded DNA fragment of between 1.5-1.6 kb. The fragment was cloned and the cloned fragment was analyzed to confirm that it did, in fact, encode the guinea pig PH-20 protein. Labeled probe was generated from this clone by conventional methods.

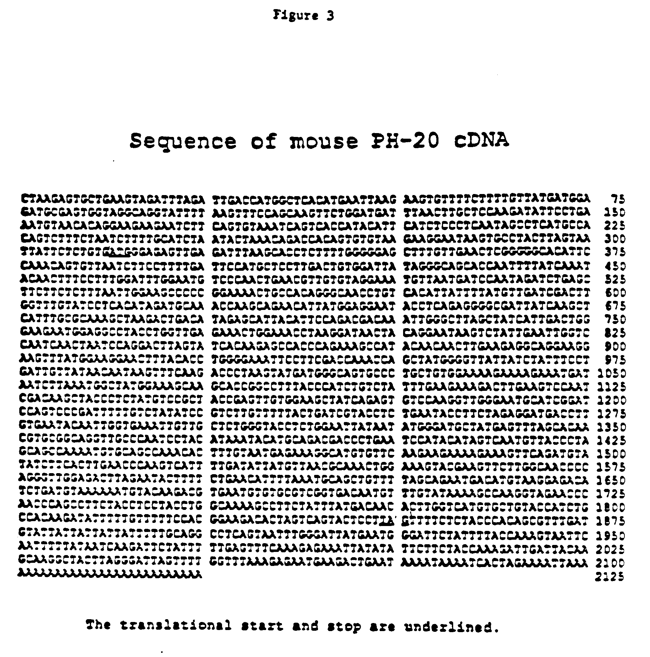

The murine cDNA library was screened using the guinea pig probe described above. Two positive clones were identified. The two clones represent about 1500 base pairs of DNA. Neither of the clones contained sequences from the 5' portion of the cDNA. Anchored PCR using a set of primers complementary to the 5' end of one of the positive clones was used to clone the 5' portion of the murine gene. The DNA sequence is set forth in Figure 3.

### Reference Example 4: Isolation of DNA Encoding Human PH-20

DNA encoding human PH-20 was isolated and cloned by screening a human testis library in lambda gt11. The library was plated at a density of about 3,000 plaques per 90 mm plate. Phage plaques were transferred to duplicate filters and screened with a mix of two radioactively labeled DNA probes, a mouse PH-20 cDNA and a guinea pig PH-20 cDNA. More specifically, the guinea pig probe was the labeled full length guinea pig PH-20 probe described above and the murine clone was one of the two murine clones which lacked sequences from the 5' end of the murine cDNA.

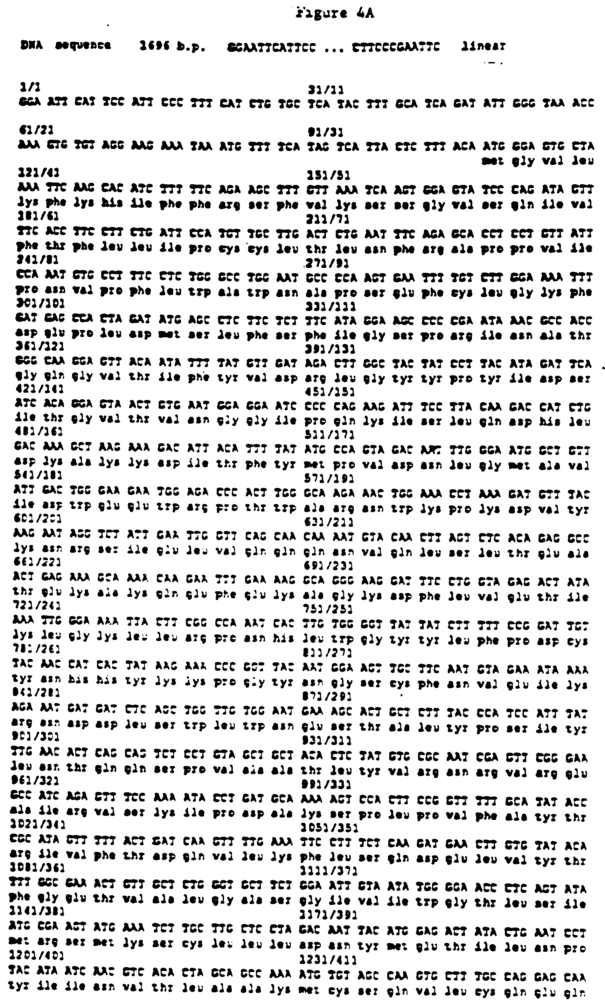

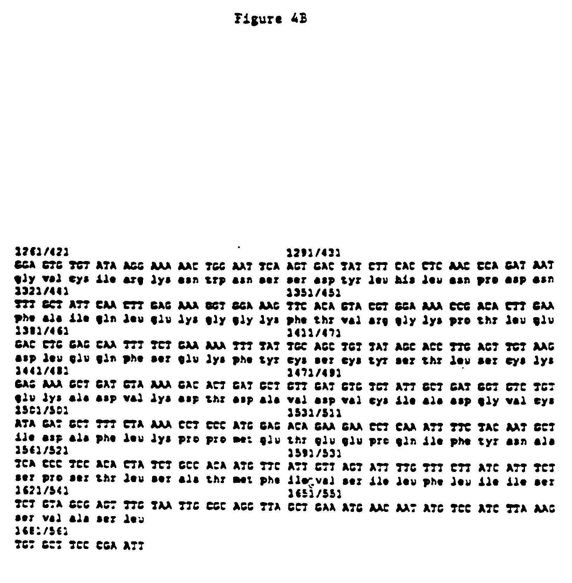

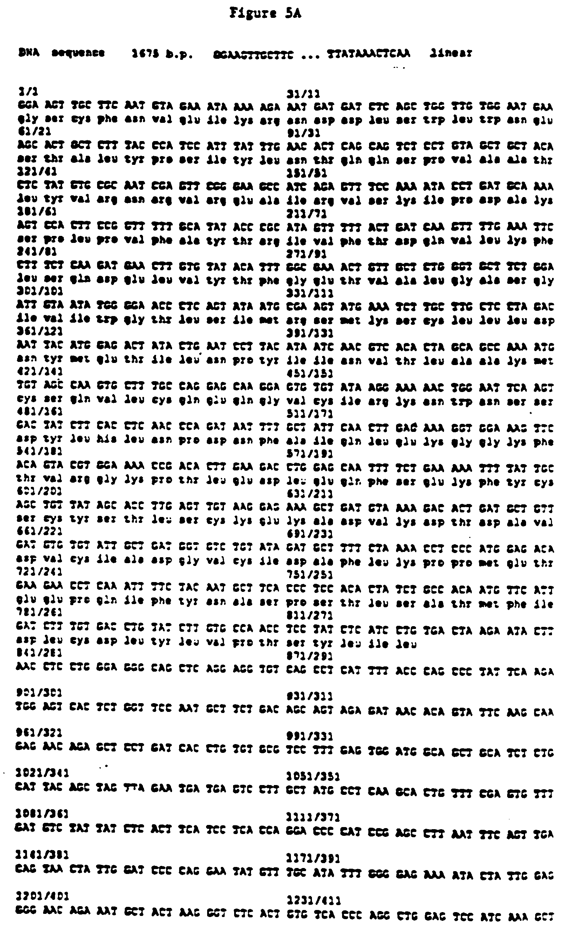

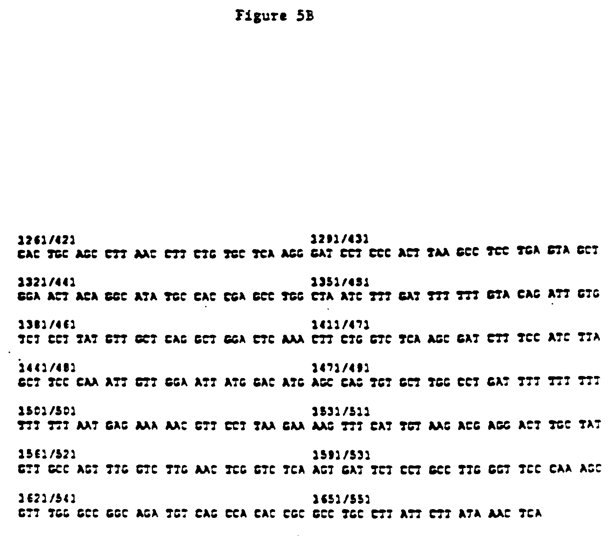

Positive plaques that hybridized with the mix of two probes were picked and purified. The cDNA inserts were subcloned and the DNA sequence determined using standard techniques. Two cDNA clones were obtained. Each of the two encode a different form of human PH-20. One human clone is designated H18 (Figure 4) and one is designated H16 (Figure 5).

H18 is a full-length clone which contains an open reading frame of 510 amino acids and short 5' and 3' untranslated regions. The protein encoded in the open reading frame of H18 is 59% identical and 74% similar (includes conservative substitutions) to guinea pig PH-20.

H16 is a partial length clone that encodes the carboxyl terminal half of human PH-20. Nucleotide 1 in H16 corresponds with nucleotide 814 in H18. The sequence of H16 from nucleotide 1-781 is identical to the sequence of H18 from nucleotide 814-1594; the sequence of H16 beginning at nucleotide 782 and continuing to nucleotide 1675 is different from the sequence of H18 beginning at nucleotide 1595 and continuing to nucleotide 1696. In terms of the encoded PH-20 protein, the partial protein encoded by H16 is identical to the protein encoded by H18 between amino acids 236-496 (amino acid numbering based on H18 sequence). H16 then encodes amino acids 497-511 and H18 encodes amino acids 497-510 and the sequences are different at each residue.

### Expression and Purification of Human PH-20

The full-length clone for PH-20 (H18) was subcloned into two E. coli expression vectors, pMAL-p and pMAL-c (New England Biolabs, Beverly, MA). In both vectors, PH-20 is made as a fusion protein, the N-terminal fusion partner being the maltose binding (MBP) protein of E. coli. In pMAL-p, the encoded MBP (which is normally a periplasmic protein) has its usual signal sequence which results in the MBP-PH-20 fusion being targeted to the periplasm. For fusion proteins that can be successfully exported to the periplasm, this location has the advantage that disulfide bonds form (twelve cysteines are present in human PH-20) yielding a potentially more immunogenic protein. In pMAL-c, the signal sequence for MBP is not present, and the fusion protein is found in the cytoplasm and does not form disulfides. Human PH-20 is produced from both pMAL-p and pMAL-c. However, in pMAL-p carrying strains, the amount of hPH-20 made is low, whereas in pMAL-c carrying strains, the amount of PH-20 made is high (the fusion protein is the major band in an E. coli extract on a Coomassie blue-stained SDS-PAGE gel). To purify the human PH-20 fusion protein, the MBP-PH-20 fusion protein is bound to an amylose resin (to which MBP binds) and eluted with maltose.

### Example 5: Isolation of DNA Encoding PH-30 Sperm Surface

This Example describes the isolation of DNA encoding the PH-30 sperm surface protein. The subject matter of this example was published by Blobel et al. (Nature 356: 248 (1992)).

The mature PH-30 protein is a dimeric protein having an α and a β subunit. The mature PH-30 protein was affinity-purified as described by Primakoff et al. (J. Cell. Biol. 104: 141-149 (1987)). Following isolation, subunits were separated by SDS-PAGE and electroeluted using a Schleicher and Schuell elutrap. Amino-terminal and internal peptide sequences were determined from PH-30 α and β subunits as they occur on fertilization-competent (mature) sperm (see Blobel et al., J. Cell Biol. 111: 69-78 (1990)).

To determine N-terminal peptide sequence, the eluted protein was reduced and purified by reverse-phase HPLC. For internal peptide sequence of the a subunit, the eluted, reduced protein was cleaved with trypsin before HPLC. Peptides were sequenced by automated Edman degradation using an Applied Biosystems gas phase sequencer.

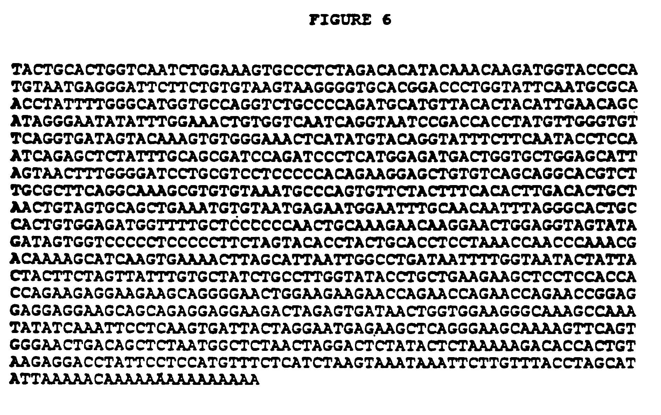

For the a subunit, the sequences determined were YCTGQSGKCPLDTYKQDG (SEQ ID NO 24) and ALFAAIQIPHGDD (SEQ ID NO 30). These two peptide sequences were used to design degenerate oligonucleotide primers for nested polymerase chain reactions (PCR) on guinea pig testis cDNA. The primer identity and combinations are specified more particularly in Blobel et al. (Nature 356:284 (1992)). The nested PCR product, a band of 308 nucleotides, was subcloned, sequenced and shown to encode the previously determined peptide sequences. The nested PACE protocol was then carried out to complete the 3' sequence of mature PH-30 α and to confirm the peptide sequence of the mature N-terminus. Amplifed DNA fragments were excised from an agarose gel, purified with Geneclean (Bio 101, Inc.), and directly sequenced. Both strands were sequenced with primers spaced at about 175 nucleotide intervals; the secondary reaction products from two different primary RACE reactions were identical. The deduced amino-acid sequences of the PCR products were found to include previously determined peptide sequences. The remainder of the a sequence was generated by a nested rapid amplification of cDNA ends (RACE) protocol (Frohman and Martin, Techniques 1: 165-170 (1989)). The deduced sequence of the PH-30 α subunit is shown in Figure 6. The nucleotide sequence of the α subunit contains one open reading frame encoding 289 amino acids.

The sequence of the β subunit was determined in a similar manner. Peptide sequences were determined as described for the α subunit but to generate N-terminal peptide sequence, the β subunit was transferred to an Immobilon (Dupont Biotechnology Systems, Boston, Massachusetts) membrane. To generate internal peptide sequence, the eluted protein was cleaved with cyanogen bromide or V8 protease, electrophoresed, and transferred to Immobilon. Alternatively, eluted protein was digested sequentially with CNBr and trypsin, followed by reverse-phase HPLC. The peptide sequences determined by these methods were SNPV_GNNRVEQGEDCDCGSQEECQDTC (SEQ ID NO 22), AQGP (SEQ ID NO 16), STDECDLPEYCNGSSGACQEDL (SEQ ID NO 16), MGSVD_FEQL_TKNDIT_N (SEQ ID NO 27), A_GASNWK (SEQ ID NO 31), M_IYA_ISG (SEQ ID NO 31), SAL__VR (SEQ ID NO 34), C_PS_VCR (SEQ ID NO 34) and VATV (SEQ ID NO 39). Peptides SNPV_GNNRVEQGEDCDCGSQEECQDTC (SEQ ID NO 22) and STDECDLPEYCNGSSGACQEDL (SEQ ID NO 16) were used to design four degenerate oligonucleotide primers. Nested PCR using combinations of the degenerate primers resulted in two strong bands of 185 and 206 base pairs. The 206-base pair fragment was excised from a polyacrylamide gel, eluted in water, precipitated, sequenced and found to encode PH-30 β peptide sequences. It was labeled with ³²P and used to probe an expression library of guinea-pig spermatogenic cell cDNA (Lathrop et al., J. Cell. Biol. 111: 2939-2941 (1990)) using high stringency hybridization and wash conditions.

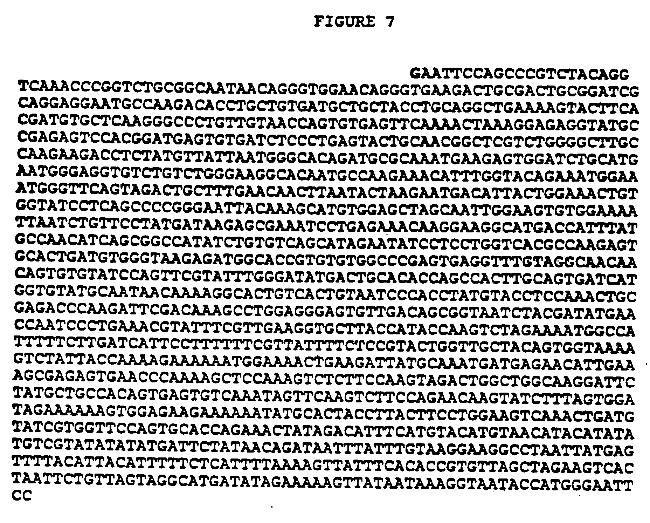

One clone, which encoded all of mature PH-30 β plus 4 amino acids of the precursor domain was identified out of 500,000 plaques and directly sequenced using PCR-amplified DNA fragments with primers spaced at about 200 nucleotide intervals. The sequence was confirmed by sequencing both strands of mature PH-30 β amplified by PCR from first strand guinea-pig testis cDNA. The deduced cDNA sequence of the PH-30 β subunit is shown in Figure 7. The nucleotide sequence of the β subunit contains one open reading frame encoding 353 amino acids.

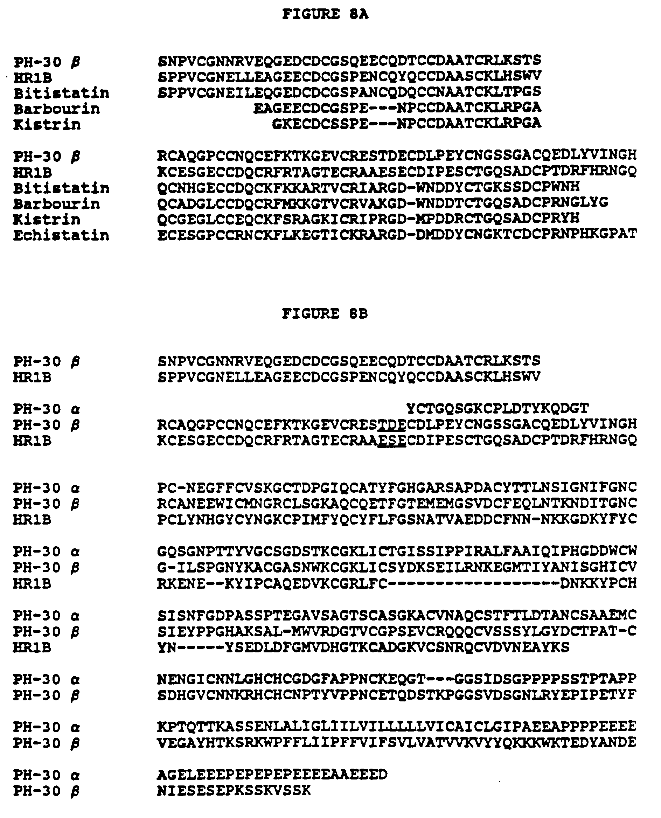

Most viral fusion proteins contain a "fusion peptide", either at the N terminus or within the polypeptide, the sequence of which is highly conserved within, but not between, virus families. Fusion peptides are (1) always located in a membrane-anchored subunit, (2) relatively hydrophobic and (3) capable of being modeled as a "sided" α helix with most of the bulky hydrophobic residues on one face. In at least one case, a helical structure is adopted when the fusion peptide interacts with a membrane surface. The hydrophobic face of the helix may mediate virus-cell membrane interactions that lead to fusion. PH-30 α contains a region (residues 90-111 in Figure 8b) that fulfills all three of these criteria of an internal fusion peptide. Putative internal viral fusion peptides have proline residues at or near their centers, similarly, this region has two prolines in the middle of its predicted helix which probably causes a kink, as do prolines in other membrane-interactive α helices. This potential fusion peptide of PH-30 α overlaps another PH-30 region (residues 82-102 in Figure 8b) that is similar in sequence to a potential fusion peptide of the E2 glycoprotein of rubella virus (GADTRCGRLICGLSTTAQYPPTR) (SEQ ID NO 42).

Most viral proteins are responsible for binding as well as fusion, to target membranes. The N-terminal 90 amino acids of mature PH-30 β contains a putative integrin-binding "disintegrin" domain that could function in the membrane-binding step that precedes sperm-egg fusion. Disintegrins such as bistatin (Shebuski et al., J. Biol. Chem. 264:21550-21556 (1989)) barbourin (Scarborough et al. J. Biol. Chem. 266:9359-9362 (1991)), kistrin (Dennis et al. Proc. Natl. Acad. Sci. 86:4022-4026 (1989)) and echistatin (Gan et al. J. Biol. Chem. 263:19832 (1988)) comprise a family of short (49-83 amino acids), soluble and highly conserved platelet aggregation inhibitors from snake venoms which act by competitive inhibition of fibrinogen binding to the integrin gpIIb/IIIa. The similarity of sperm PH-30 β to these integrin ligands suggests that it binds to a receptor, probably an integrin, on the egg plasma membrane.

Most disintegrins contain an integrin consensus binding sequence RGD. Changes in this sequence alter either the affinity (Garsky et al., Proc. Natl. Acad. Sci. 86:4022-4026 (1989)), or the specificity of binding (Scarborough et al., J. Biol. Chem. 266:9359-9362 (1991), Phillips et al., Cell 65:359-362 (1991)). Half of the known integrin ligands, however, either do not contain this tripeptide or can interact with integrins using non-RGD sequences. Nuclear magnetic resonance analysis revealed that the RGD sequences of kistrin and echistatin are at the tip of a loop where they are thought to assume a conformation necessary for high-affinity binding. If the disintegrin domain of PH-30 β adopts a similar tertiary structure, the sequence TDE and an additional (odd-numbered) cysteine would occupy the tip of such a loop and might then interact with an integrin on the egg plasmal membrane. The disintegrin domain of the longer snake venom protein HR1B contains the sequence ESE and an additional cysteine in this position.

Two lines of indirect evidence support the hypothesis that PH-30 β binds to an egg plasma membrane integrin: proteolysis of mouse eggs reduces their fertilization competence (Calarco Microscope Tech. 17:401-411 (1991), Boldt et al., Gamete Res. 23:91-101 (1989)), and two of the implicated egg surface polypeptides have a molecular weight of 95K and 150K, similar to the α and β integrin subunits; also micromolar concentrations of RGD-containing peptides inhibit fusion between human or hamster sperm and hamster eggs lacking a zona pellucida. RGD peptides might compete with PH-30 homologues on these sperm, even if the homologues do not contain an RGD sequence, as RGD peptides can compete for binding of non-RGD integrin ligands.

### Example 6: Contraceptive Vaccination by the Administration of PH-30 Protein

The identification and purification of the guinea pig sperm surface protein PH-30 has been described by Primakoff et al. (J. Cell. Biol. 104: 141 (1987)). This Example describes the results of experiments in which both male and female guinea pigs were immunized with purified PH-30 protein, and the effect of this immunization on fertility.

Female or male Hartley guinea pigs (about 300 grams (females) or 600-650 grams (males) at the time of first injection) received two injections of PH-30 in the amounts stated below. PH-30, purified from sperm by mAb-affinity chromatography, showed no detectable contaminants using silver-staining of purified protein on SDS gels. Purity of each PH-30 preparation used for immunization of females or males was verified by SDS polyacrylamide gel electrophoresis and silver staining. The affinity-purified PH-30, in 0.375 ml phosphate-buffered saline (PBS) containing 3 mM octyl glucoside (OG) was emulsified with 0.375 ml complete Freund's adjuvant (CFA). Each animal received 0.5 ml of the emulsion subcutaneously in the back and 0.25 ml intramuscularly in a rear leg. About 1 month later the same amount of PH-30 in PBS and 3 mM OG was emulsified with incomplete Freund's adjuvant (IFA), and was injected in the same sites in each animal (the following exceptions apply: males 1 and 3 received only one injection). Control females and males received the same injections on the same schedule and containing PBS and 3 mM OG and CFA or IFA, but lacking PH-30. To allow the injected females to mate, about two months after the initial injection they were housed with males for 3 weeks. Each cage contained one male (575-600g), one PH-30 immunized female, and from 2-4 control injected females. After three weeks, the females were separated from the males, pregnant females had litters and progeny were counted. Control-injected females that failed to become pregnant had been in cages where the other controls did become pregnant, indicating that all the males mating with immunized females were fertile. To allow the injected males to mate, about four months after the initial injection, each injected male was housed with two females (about 600 grams) for three weeks. The females and males were then separated and after an additional 5 weeks

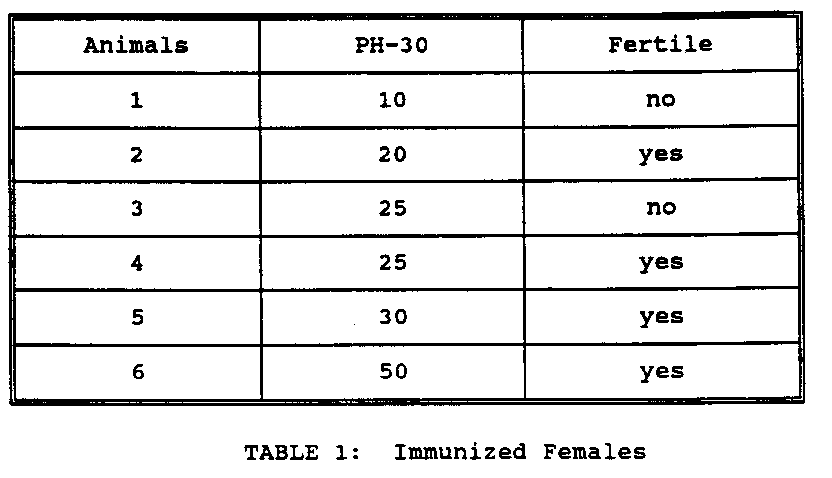

Table 1 shows that of the 6 female guinea pigs which were immunized, four were fertile. Of the control-injected females, 34 of 36 were fertile.

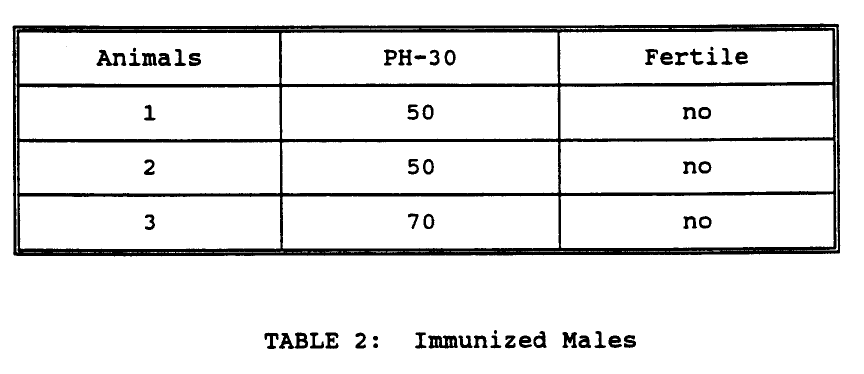

Table 2 shows that of the 3 males guinea pigs which were immunized, all 3 were infertile. Of the control-injected males, 5 out of 5 were fertile. females were killed and fetuses counted. The results of these experiments are set forth in the tables which follow.

### Example 7: Use of PH-30 Disintegrin Peptides as Inhibitor of Sperm Fusion to Egg Plasma Membrane

Peptides from the PH-30 β disintegrin domain were tested for inhibition of sperm binding to the egg plasma membrane. More specifically, two modified PH-30 β peptides were tested. These two peptides contain the sequence TDE which is in the same position in the disintegrin domain as RGD, the active tripeptide in most snake disintegrins. The two modified PH-30 peptides tested were STDECDLK (SEQ ID NO 43) and CSTDEC (SEQ ID NO 44). The first of the two peptides is a linear peptide and was modified by the addition of a lysine residue (K) to facilitate conjugation of the peptide to an agarose bead. The second of the two peptides is a cyclized peptide and was modified by the addition of a cysteine residue (C) to allow cyclization by disulfide formation. Two control peptides, which represent a random string of amino acids, were also included in the fusion inhibition assay. The control peptides were GRGDTP (SEQ ID NO 45) and GRGES (SEQ ID NO 46).

The fusion inhibition assay was carried out as follows. Eggs were collected from guinea pig ovaries. The eggs were incubated in tissue culture medium overnight. The zona pellucida was removed with a mixture of proteases. The zona pellucida free eggs were incubated in culture media with peptide at a specified concentration for 30 minutes.

Sperm collected from the epididymis of male guinea pigs was capacitated by incubation and acrosome reacted as described by Fleming and Yanagimachi (Gamete Res. 4: 253-273 (1981)) and added to the eggs and incubated for 15 minutes. The eggs were then transferred to a sperm free culture medium and incubated for an additional 1 hour and 45 minutes. The eggs were then fixed and stained as described by Primakoff et al. (J. Cell. Biol. 104: 141 (1987)). The total number of swollen sperm heads were then counted. Swollen sperm heads are an indication that the sperm and egg have fused.

On the basis of these observations, several indices are calculated. The fertilization index (F.I.) is determined by dividing the total number of swollen heads by the total number of eggs. The fertilization rate (F.R.) is the percentage of eggs fertilized. The percent inhibition is determined by dividing the fertilization index of the experimental peptide by the fertilization index of the control peptide.

The data obtained from the sperm-egg fusion inhibition assay are summarized in Tables 3-6 below. The results of these experiments show clearly that compared to no peptide, incubation of the zona pellucida free eggs with the control peptides GRGES and GRGDTP results in no significant inhibition of sperm-egg fusion. The fusion rates observed between the no peptide assay and the control peptide assay were similar or were higher in the presence of control peptide (Table 3).

However, when experimental peptides CSTDEC and STDECDLK were added, sperm-egg fusion was strongly inhibited compared to no peptide addition (Table 4) or compared to control peptide addition (Table 5). When the fertilization index is near the biologically normal level of 1 sperm per egg, STDECDLK inhibits sperm-egg fusion 92% and CSTDEC inhibits 89% (Table 4). It can be concluded from these data that the PH-30 β disintegrin domain represents an epitope which is critical in sperm-egg fusion. Antibodies which bind specifically to this epitope would be predicted to block sperm/egg fusion.

**TABLE 3**

| INABILITY OF A CONTROL PEPTIDE (GRGES) TO INHIBIT SPERM-EGG FUSION | | | |
|---|---|---|---|
| Group | #Eggs | F.I. | F.R. |
| GRGES | 14 | 2.3 | 93 |
| control | 14 | 1.8 | 64 |
| | | | |
| GRGES | 9 | 5.3 | 100 |
| control | 17 | 2.2 | 82 |
| | | | |
| GRGES | 12 | .92 | 75 |
| control | 7 | 1.6 | 86 |
| | | | |

**TABLE 4**

| COMPARISON OF TDE PEPTIDES AND NO PEPTIDE CONTROL IN THE SPERM-EGG FUSION ASSAY | | | | |
|---|---|---|---|---|
| Group | # Eggs | F.I. | F.R. | % Inhibition |
| control | 12 | 1.08 | 75 | |
| STDECDLK | 11 | .09 | 9 | 92 |
| | | | | |
| control | 11 | 3.8 | 91 | |
| STDECDLK | 11 | 1.9 | 73 | 50 |
| | | | | |
| control | 9 | 1.78 | 100 | |
| STDECDLK | 14 | .57 | 29 | 68 |
| | | | | |
| control | 16 | 1.6 | 63 | |
| CSTDEC | 12 | .17 | 8 | 89 |
| | | | | |
| control | 17 | 2.2 | 82 | |
| CSTDEC | 9 | .11 | 11 | 95 |

**TABLE 5**

| COMPARISON OF A CONTROL PEPTIDE (GRGES), TWO TDE PEPTIDES AND A RGD PEPTIDE IN SPERM-EGG FUSION ASSAY | | | | |
|---|---|---|---|---|
| GROUP | # Eggs | F.I. | F.R. | % Inhibition |
| GRGES | 14 | 2.4 | 64 | |
| CSTDEC | 8 | .25 | 25 | 90 |
| | | | | |
| GRGES | 9 | 5.3 | 100 | |
| CSTDEC | 9 | .11 | 11 | 98 |
| | | | | |
| GRGES | 10 | 1.1 | 60 | |
| STDECDLK | 8 | .25 | 25 | 77 |
| | | | | |
| GRGES | 12 | .91 | 75 | |
| GRGDTP | 12 | 1.6 | 83 | 0 |
| | | | | |

### Equivalents

Those skilled in the art will recognize, or be able to ascertain using no more than routine experimentation, many equivalents to the specific embodiment of the invention described specifically herein. Such equivalents are intended to be encompassed in the scope of the following claims.

## Claims

1. Use of an isolated, immunogenic portion of PH-30 sperm surface protein in a method of contraception comprising administering an effective amount of the peptide to a mammal, the peptide stimulating antibodies in the mammal which bind to the PH-30 sperm surface protein in vivo thereby preventing or substantially reducing the rate of sperm-egg fusion.

2. Use of an isolated, immunogenic portion of PH-30 sperm surface protein for the manufacture of a composition for the prevention of pregnancy by administration of the peptide to a mammal to effect the stimulation of antibodies which bind to the PH-30 sperm surface protein.

3. The use of Claim 1 or Claim 2, wherein the peptide is homologous to an integrin-binding domain.

4. A contraceptive composition comprising an essentially pure peptide having an amino acid sequence which is homologous to that of an immunogenic epitope of PH-30 sperm surface protein, in a pharmaceutically acceptable carrier.

5. The contraceptive composition of Claim 4, wherein the essentially pure peptide is produced by expressing DNA encoding an immunogenic epitope of the PH-30 sperm surface protein in a recombinant DNA expression vector, and the peptide may be homologous to an integrin binding domain of the PH-30 sperm surface protein.

6. A contraceptive composition comprising a portion of PH-30 sperm surface protein of a mammal which is effective for the stimulation of antibodies which bind to the PH-30 sperm surface protein.

7. The contraceptive composition of Claim 6, wherein the portion is substantially identical in amino acid sequence to that of a PH-30 integrin-binding domain.

8. Use of a purified PH-30 sperm surface protein in the manufacture of a composition which stimulates antibodies in a mammal which antibodies bind to PH-30 sperm surface proteins.

9. Use of a purified PH-30 sperm surface protein in the manufacture of a composition for use in a mammal to immunise against PH-30 sperm surface protein.

10. The contraceptive composition according to any one of claims 4-6 for use as a contraceptive in a male mammal.

11. The use according to claim 8 or claim 9 in which the composition is for use as a contraceptive in a male mammal.

## Patentansprüche

1. Verwendung eines isolierten, immunogenen Teils vom Sperma-Oberflächenprotein PH-30 in einem Verfahren zur Kontrazeption, das Verabreichen einer wirksamen Menge des Peptids an einen Säuger umfaßt, wobei das Peptid in dem Säuger Antikörper stimuliert, die an das Sperma-Oberflächenprotein PH-30 in vivo binden, wobei dadurch die Sperma-Ei-Fusion verhindert oder die Rate der Sperma-Ei-Fusion wesentlich reduziert wird.

2. Verwendung eines isolierten, immunogenen Teile vom Sperma-Oberflächenprotein PH-30 zur Herstellung einer Zusammensetzung zur Verhütung einer Schwangerschaft durch Verabreichung des Peptids an einen Säuger, um die Stimulierung von Antikörpern zu bewirken, die an das Sperma-Oherflächenprotein PH-30 binden.

3. Die Verwendung nach Anspruch 1 oder Anspruch 2, worin das Peptid zu einer Integrin-bindenden Domäne homolog ist.

4. Eine kontrazeptive Zusammensetzung, die ein im wesentlichen reinem Peptid mit einer Aminosäure-Sequenz, die zu der eines immunogenen Epitops vom Sperma-Oberfächenprotein PH-30 hamolog ist, in einem pharmazeutisch geeigneten Träger umfaßt.

5. Die kontrazeptive Zusammensetzung nach Anspruch 4, worin das im wesentlichen reine Protein durch Expression einer DNS hergestellt ist, die ein immunogenes Epitop des Sperma-Oberflächenproteins PH-30 codiert in einem rekombinanten DNS-Expressionsvektor, und das Peptid zu einer Integrin bindenden Domäne des Sperma-Oberflächenproteins PH-30 homolog sein kann.

6. Eine kantrazeptive Zusammensetzung, die einen Teil von Sperma-Oberflächenprotein PH-30 eines Säugers umfaßt, das für die Stimulierung von Antikörpern, die an das Sperma-Oberflächenprotein PH-30 binden, wirksam ist.

7. Die kontrazeptive Zusammensetzung nach Anspruch 6, worin der Teil in der Aminosäure-Sequenz zu der einer PH-30 Integrin-bindenden Domäne im wesentlichen identisch ist.

8. Verwendung eines gereinigten Sperma-Oberflächenproteins PH-30 bei der Herstellung einer Zusammensetzung, die in einem Säuger Antikörper stimuliert, und diese Antikörper an PH-30 Sperma-Oberflächenproteine binden.

9. Verwendung eines gereinigten Sperma-Oberflächenproteins PH-30 bei der Herstellung einer Zusammensetzung zur Verwendung in einem Säuger, um gegen Sperma-Oberflächenprotein PH-30 zu immunisieren.

10. Die kontrazeptive Zusammensetzung gemäß einem der Ansprüche 4 bis 6 zur Verwendung als ein Kontrazeptivum in einem männlichen Säuger.

11. Die Verwendung gemäß Anspruch 8 oder Anspruch 9, wobei die Zusammensetzung als ein Kontrazeptivum in einem männlichen Säuger verwendet wird.

## Revendications

1. Utilisation d'une portion immunogène Isolée de la protéine de surface de sperme PH-30 dans un procédé de contraception comprenant l'administration d'une quantité efficace du peptide à un mammifére, le peptide stimulant chez le mammifère des anticorps qui se lient à la protéine de surface de sperme PH-30 in vivo, prévenant ainsi ou réduisant substantiellement la fusion sperme-ovule.

2. Utilisation d'une portion immunogène isolée de la protéine de surface de sperme PH-30 pour la fabrication d'une composition pour prévenir une grossesse par administration du peptide chez un mammifère de manière à stimuler des anticorps qui se lient à la protéine de surface de sperme PH-30.

3. Utilisation selon la revendication 1 ou la revendication 2, dans laquelle le peptide est homologue à un domaine de liaison à une intégrine.

4. Composition contraceptive comprenant un peptide essentiellement pur ayant une séquence d'acides aminés qui est homologue de celle d'un épitope immunogène de protéine de surface de sperme PH-30, dans un véhicule pharmaceutiquement acceptable.

5. Composition contraceptive selon la revendication 4, dans laquelle le peptide essentiellement pur est produit par expression de l'ADN codant pour un épitope immunogène de la protéine de surface de sperme PH-30 dans un vecteur d'expression d'ADN recombinant, le peptide pouvant être homologue à un domaine de liaison à une intégrine de la protéine de surface de sperme PH-30.

6. Composition contraceptive comprenant une portion de la protéine de surface de sperme PH-30 d'un mammifère qui est efficace pour la stimulation d'anticorps qui se lient à la protéine de surface de sperme PH-30.

7. Composition contraceptive selon la revendication 6, dans laquelle la séquence d'acides aminés de la portion est essentiellement identique à celle d'un domaine de liaison à une intégrine PH-30.

8. Utilisation d'une protéine de surface de sperme PH-30 purifiée pour la fabrication d'une composition qui stimule les anticorps chez un mammifère, lesdits anticorps se liant aux protéines de surface de sperme PH-30.

9. Utilisation d'une protéine de surface de sperme PH-30 purifiée pour la fabrication d'une composition destinée à immuniser un mammifère contre la protéine de surface de sperme PH-30.

10. Composition contraceptive selon l'une quelconque des revendications 4-6, pour une utilisation comme contraceptif chez un mammifère mâle.

11. Utilisation selon la revendication 8 ou la revendication 9, dans laquelle la composition est destinée à une utilisation comme contraceptif chez un mammifère mâle.
